# EUROPEAN PATENT APPLICATION

(11) **EP 4 506 341 A1**
(43) Date of publication of application: **12.02.2025**
(21) Application number: 23382823.5
(22) Date of filing: 07.08.2023
(51) Int. Cl.: C07D 295/16, C07C 233/58, A61K 31/5375, A61K 31/54, A61P 17/02, A61P 41/00

(54) **TISSUE REGENERATION APPROACHES**

(71) Applicant: ZeCardio Therapeutics SL, 08916 Barcelona (ES); ZeClinics SL, 08916 Barcelona (ES); Taros Chemicals GmbH & Co. KG, 44227 Dortmund (DE); Universität Bern, 3012 Bern (CH); Centro Nacional de Investigaciones Cardiovasculares Carlos III (F.S.P.), 28029 Madrid (ES)
(72) Inventor: Terriente Felix, Francisco Javier, 08916 Barcelona (ES); Jung, Carole Veronique, 08916 Barcelona (ES); D'Amico, Davide, 08916 Barcelona (ES); Muriel Moreno, Beatriz, 08916 Barcelona (ES); Di Donato, Vincenzo, 08916 Barcelona (ES); Calzolari, Simone, 08916 Barcelona (ES); Sahun Abizanda, Ignacio, 08916 Barcelona (ES); Levi, Laura Mariana, 44227 Dortmund (DE); Ajani, Haresh, 44227 Dortmund (DE); Corpet, Martin, 44227 Dortmund (DE); Mercader Huber, Nadia, 3012 Bern (CH); Sanz-Morejón, Andrés, 3012 Bern (CH); Sánchez-Iranzo, Héctor, 28029 Madrid (ES)
(74) Representative: Hoffmann Eitle

(57) **Abstract**

The invention provides a compound of formula (I) solvate, stereoisomer or salt thereof, wherein R₁ represents H; (C₁-C₁₀)alkyl optionally substituted; (C₂-C₁₀)alkenyl optionally substituted; or (C₂-C₁₀)alkynyl optionally substituted; R₂ₐ to R₂ₑ are the same or different and represent hydrogen, hydroxyl, or halogen; R₃ and R₄ are the same or different and represent (C₁-C₁₀)alkyl optionally substituted; (C₂-C₁₀)alkenyl optionally substituted; (C₂-C₁₀)alkynyl optionally substituted; or, alternatively, R₃ and R₄ form, together with the N heteroatom to which they are attached, a 5-6 membered heterocycle including a second heteroatom selected from N, O or S.

The invention further provides compositions and uses in the regeneration and repairing of a tissue.

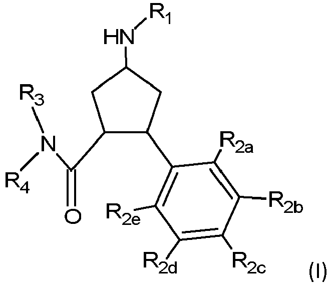

## Description

### FIELD OF THE INVENTION

The present invention refers generally to compounds, compositions and their use in methods for the repair or regeneration of damaged or diseased cells or tissue, particularly in the repair and/or regeneration of cardiac tissue.

### BACKGROUND

Many diseases, injuries and maladies involve loss of or damage to cells and tissues.

Examples include, but are not limited to neurodegenerative disease, endocrine diseases, cancers, and cardiovascular disease.

These non-limiting examples are the source of substantial medical costs, reduced quality of life, loss of productivity in workplaces, workers compensation costs, and of course, loss of life. Cardiovascular diseases (CVDs) are the leading cause of mortality globally, taking an estimated 17.9 million lives each year and causing the death of over 3,9 millions individuals in Europe. 85% of these deaths are due to heart attack and stroke according to the World Health Organization. Myocardial infarction or heart attack is the prevalent cause of cardiovascular diseases with a prevalence of 3,8% in the population under 60 years old worldwide (Salari et al., 2023). Even among those who survive the MI, many will still die within one year, often due to reduced cardiac function, associated side effects, or progressive cardiac disease.

Heart disease is the leading cause of death for both men and women, and coronary heart disease, the most common type of heart disease, led to approximately 400,000 deaths in 2008 in the US. Regardless of the etiology, most of those afflicted with coronary heart disease or heart failure have suffered permanent heart tissue damage, which often leads to a reduced quality of life.

The discovery of new therapeutic targets and treatments is necessary to overcome drug attrition associated with myocardial infarction and heart failure. Current treatments target mostly the symptoms associated with heart failure such as high blood pressure, cardiac load and cardiac contractility but do not tackle specifically heart regeneration and repair of the injured heart (Heallen et al., 2019). Stem cells therapy is thought to have a great potential to increase cardiac function. However, clinical trials have displayed uncertain benefits of transplanting stem cells after myocardial infarction, while posing high risk of tumorigenesis (Carbone et al., 2021).

There exists a need for methods and compositions to repair and/or regenerate tissue that has been damaged (or is continuing to undergo damage) due to injury, disease, or combinations of causes. The past two decades have seen a strong decrease in the development of cardiovascular drugs and have mostly focused on the development and improvement of drugs targeting known processes. The ever growing burden of heart failure and the current unmet need for new drugs make discovery of new cardiovascular and tissue regeneration therapies essential (Hong, 2022).

### SUMMARY OF THE INVENTION

White classical therapies such as pharmacological intervention or device-based intervention or surgery provide positive effects, there are provided herein compounds and compositions that yield unexpectedly beneficial effects in the repair or regeneration of tissues, particularly of damaged or injured tissues.

The present inventors provide new compounds of formula (I) which can be used as TOB1 inhibitors.

Unexpectedly, the present inventors have found, for the first time, that TOB1 inhibition provides a positive effect on mammal tissue regeneration.

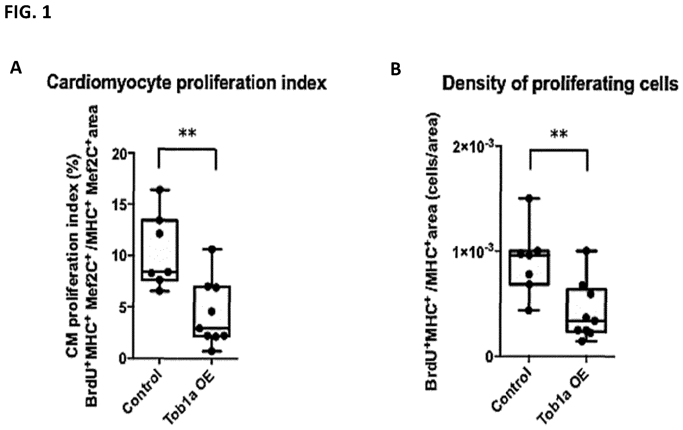

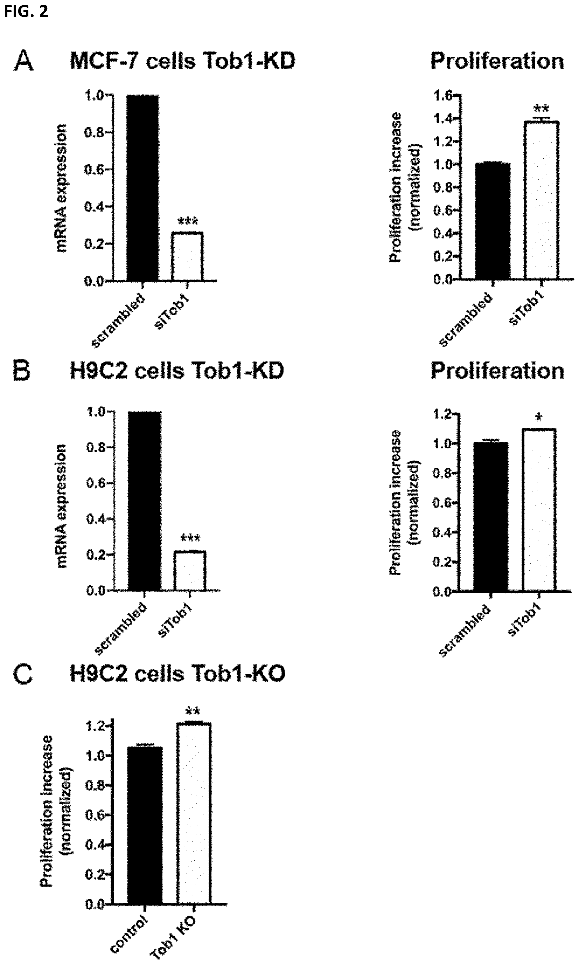

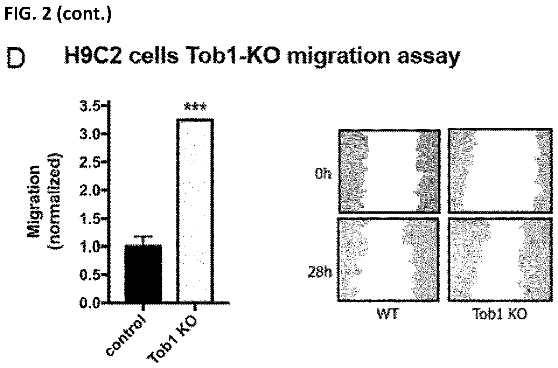

As it is shown below, the inventors firstly concluded that *tob1* repression was necessary to promote cardiomyocyte proliferation (Fig. 1 and Fig. 2).

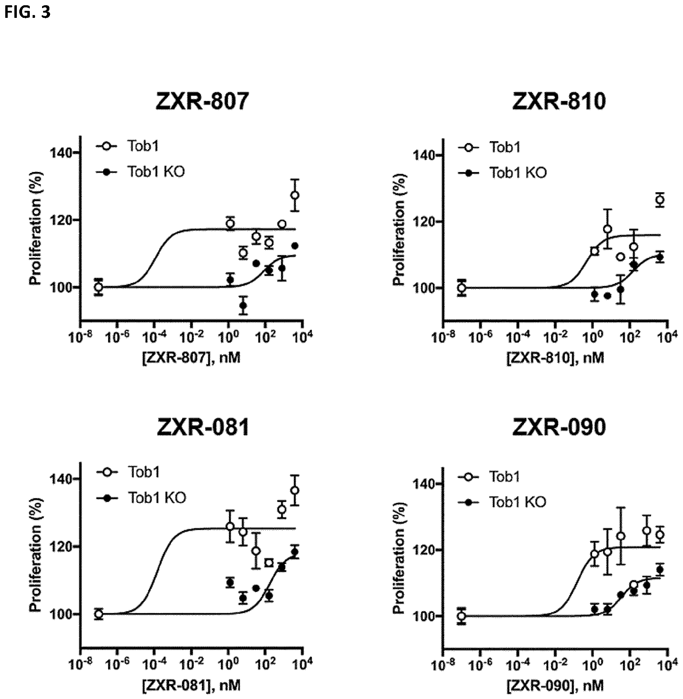

With further experimental research they confirmed that such effect on proliferation was not exclusive to cardiomyocytes and that it could be extrapolated to epithelial tissues. As shown below, using two different cell lines, MCF7 (which validated Tob1 as relevant in human epithelial tissues), and H9C2 (which validated that Tob1 played a role in a cell line derived from cardiac tissue), they found that Tob1 function depletion, either by siRNA knockdown or by CRISPR/Cas9 knockout, promoted an increase in proliferation (Fig. 3A-C). Therefore, these data not only support the involvement of TOB1 inhibition in regenerating cardiac tissue, but also in regenerating other tissues.

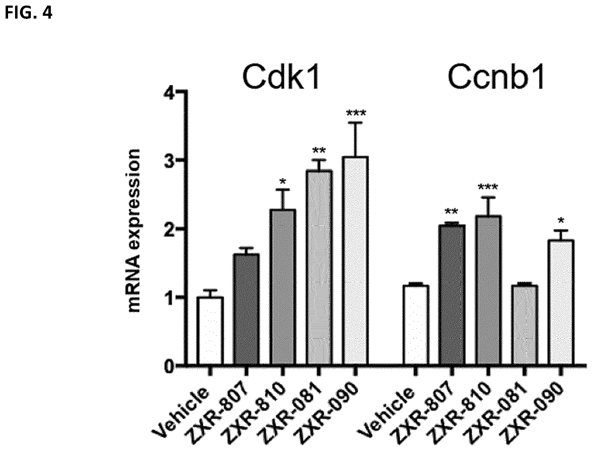

The experimental data provided below also show that the compounds of formula (I) provided by the present invention are able to efficiently inhibit Tob1. In fact, Fig. 4 and Table 2 provide data supporting the remarkably high selectivity against Tob1. Paying attention to Table 2, one can observe that the EC50 value of the compounds of the invention when added to the wild type H9C2 cell line (cardiomyoblast cell line expressing tob1) was substantially lower (at least 200-fold lower) than the H9C2 cell line lacking Tob1 gene.

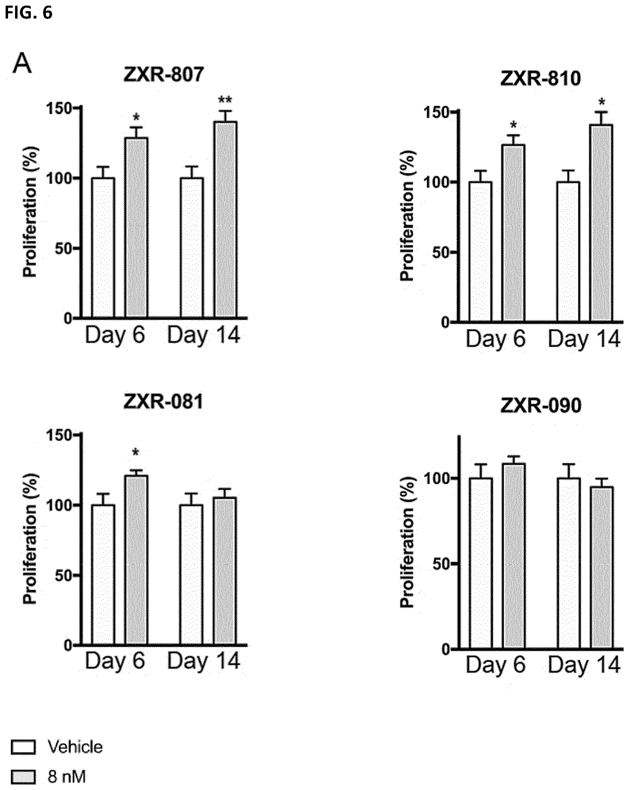

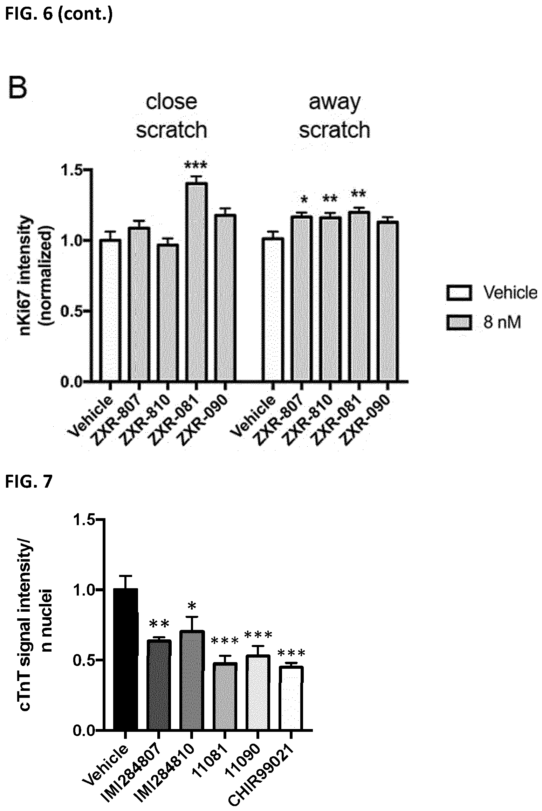

The compounds of the invention not only provide a strong capacity to promote cellular proliferation, but also provide a remarkable increase in cell migration (Fig. 6). This is an important observation, given that renewal of lost or damaged tissue relies not only on cell division but also on the migration of de novo produced cells to the site of injury.

Altogether the compounds of the invention can mean a great advance in the regeneration of tissues due to the remarkable increase in cell proliferation and migration due to the selective and potent inhibition of TOB1.

The present invention provides a compound of formula (I), a stereoisomer, solvate or salt thereof: 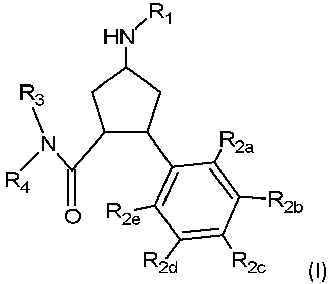 wherein:
R₁ represents H; (C₁-C₁₀)alkyl; (C₁-C₁₀)alkyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; (C₂-C₁₀)alkenyl; (C₂-C₁₀)alkenyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; (C₂-C₁₀)alkynyl; (C₂-C₁₀)alkynyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; -C(O)Rs, and -C(O)OR₆;
R₂ₐ to R₂ₑ are the same or different and represent hydrogen, hydroxyl, or halogen;
R₃ and R₄ are the same or different and represent (C₁-C₁₀)alkyl; (C₁-C₁₀)alkyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; (C₂-C₁₀)alkenyl; (C₂-C₁₀)alkenyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; (C₂-C₁₀)alkynyl; (C₂-C₁₀)alkynyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; or, alternatively,
R₃ and R₄ form, together with the N heteroatom to which they are attached, a 5-6 membered heterocycle including a second heteroatom selected from N, O or S;
R₅ and R₆ represent H; (C₁-C₁₀)alkyl; (C₁-C₁₀)alkyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; (C₂-C₁₀)alkenyl; (C₂-C₁₀)alkenyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; (C₂-C₁₀)alkynyl; (C₂-C₁₀)alkynyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; and
"heterocycle" means a ring system having one or two rings, each one of the rings:
   - being saturated, partially saturated or aromatic, and
   - consisting of 5 or 6 members, wherein one of the members corresponds to the N atom to which R₃ and R₄ are attached, and the remaining members are selected from CRₓ, C(Rₓ)₂, NR_{y}, N, O, or S; Rₓ is selected from hydrogen, (C₁-C₁₀)alkyl optionally substituted with one or more Z substituents; (C₂-C₁₀)alkenyl optionally substituted with one or more Z substituents; or (C₂-C₁₀)alkynyl optionally substituted with one or more Z substituents; and R_{y} is selected from hydrogen; (C₁-C₁₀)alkyl optionally substituted with one or more Z substituents, (C₂-C₁₀)alkenyl optionally substituted with one or more Z substituents, or (C₂-C₁₀)alkynyl optionally substituted with one or more Z substituents; and Z is selected from the group consisting of: (C₁-C₁₀)alkyl; -O-(C₁-C₁₀)alkyl; (C₁-C₁₀)haloalkyl; -O-(Cₐ-C₁₀)haloalkyl; (C₂-C₁₀)alkenyl; (C₂-C₁₀)alkynyl; and halogen.

Advantageously, the compounds of the invention are small molecules in nature, being of low molecular weight, that can readily cross cell membranes to reach the target . And consequently, they provide further advantages, among others: they are less invasive; deleterious immune responses are minimized; and the predictability of pharmacokinetics is higher. Altogether, small molecules provide a better dosing protocol for patients, and a much more cost effective drug development.

In a second aspect the present invention provides a pharmaceutical composition comprising the compound of formula (I), solvate, stereoisomer or salt thereof, as defined in the present invention, together with one or more pharmaceutically acceptable excipients and/or carriers.

In a third aspect the present invention provides a topical cosmetic composition comprising the compound of formula (I), solvate, stereoisomer or salt thereof, as defined in the present invention, together with one or more cosmetically acceptable excipients and/or carriers.

In a fourth aspect the present invention provides the use of a compound of formula (I), solvate, stereoisomer or salt thereof, according to the present invention, for use in therapy.

In a fifth aspect the present invention provides a compound of formula (I), solvate, stereoisomer or salt thereof, according to the present invention for use in repairing or regenerating a cell tissue, particularly cardiac cell tissue. This aspect can be formulated as the compound of formula (I), solvate, stereoisomer or salt thereof, according to the present invention, for the manufacture of a medicament for repairing or regenerating a cell tissue. This aspect can alternatively be formulated as a method for the repairment or regeneration of a cell tissue, the method comprising the step of administering a therapeutically effective amount of a compound of formula (I), solvate, stereoisomer or salt thereof, or of the pharmaceutical composition, as provided by the invention, to a subject in need thereof.

In a sixth aspect the present invention provides a TOB1 inhibitor for use in the prevention, repairment or regeneration of a cardiac tissue injury in a mammal by inducing the proliferation and/or migration of cardiomyocytes. This aspect can alternatively be formulated as the use of a TOB1 inhibitor for the manufacturer of a medicament for preventing, repairing or regenerating a cardiac tissue injury in a mammal by inducing the proliferation and/or migration of cardiomyocytes. This aspect can also be alternatively formulated as a method for the prevention, repairment or regeneration of a cardiac tissue injury in a mammal, the method comprising administering a therapeutically effective amount of a TOB1 inhibitor to a subject in need thereof.

In a seventh aspect the present invention provides the cosmetic non-therapeutic use of a compound of formula (I), solvate, stereoisomer or salt thereof, according to the first aspect of the invention, or of the topical cosmetic composition according to the third aspect of the invention, for the skin regeneration or repairment of skin damage. This aspect can alternatively be formulated as a cosmetic method for skin regeneration or repairment, the method comprising applying topically an amount of a compound of formula (I), solvate, stereoisomer or salt thereof, or of the cosmetic composition, as provided by the invention, to a subject in need thereof.

### BRIEF DESCRIPTION OF DRAWINGS

Fig. 1. Tob1 overexpression inhibits cardiac regeneration in zebrafish. (A) Cardiomyocyte proliferation index in injured hearts of control adult zebrafish (n = 7) and overexpressing Tob1 (n = 9), evaluated by the number of proliferating (BrdU+) cardiomyocytes (MHC+, Mef2C+) per myocardial area (total cardiomyocytes) (B). Shown are individual values in box and whisker plots. Asterisks indicate ** P<0,01 according to unpaired two-tailed Student's t-test.
Fig. 2. Loss of Tob1 promotes cell proliferation and cell migration in MCF-7 and H9C2 cells. (A, B) 75-80% knockdown of Tob1 expression produces an increase in proliferation in MCF-7 (A) and in H9C2 cells (B) as assessed by the MTT assay which measures the metabolic activity of the cells and is directly correlated with the number of cells in the sample. C, D) Tob1 knockout H9C2 cells generated by CRISPR/Cas9 gene edition show increased proliferation (C) and migration (D). Shown are data means normalized to control ± SEM. Asterisks indicate *** P<0,001; ** P<0,01 and *P<0,05 according to unpaired two-tailed Student's t-test.
Fig. 3. Proliferation dose response curves of the parental compound ZXR-807 and its chemical analogues ZXR-810, ZXR-081 and ZXR-090 in control H9C2 cells and Tob1 knockout (KO) cells. Proliferation was measured by MTT assay in response to increasing concentrations of the compounds. Shown are data means normalized to control ± SEM. Data points were fitted with a nonlinear regression model (using GraphPad Prism 8) to calculate corresponding EC50 values, as shown in table 2.
Fig. 4. Transcriptional activation of proliferation marker genes by Tob1 inhibitors in H9C2 cells. Real time PCR of proliferation marker genes Cdk1 and Ccnb1 in response to 100 nM Tob1 inhibitors ZXR-807, ZXR-810, ZXR-081 and ZXR-090 in H9C2 cells. Shown are histograms with fold changes in mRNA expression levels in control and treated cells after normalization to the vehicle-treated cells. Shown are data means ± SEM. Asterisks indicate *** P<0,001; ** P<0,01 and *P<0,05 according to one-way ANOVA followed by Dunnett's multiple comparison test.

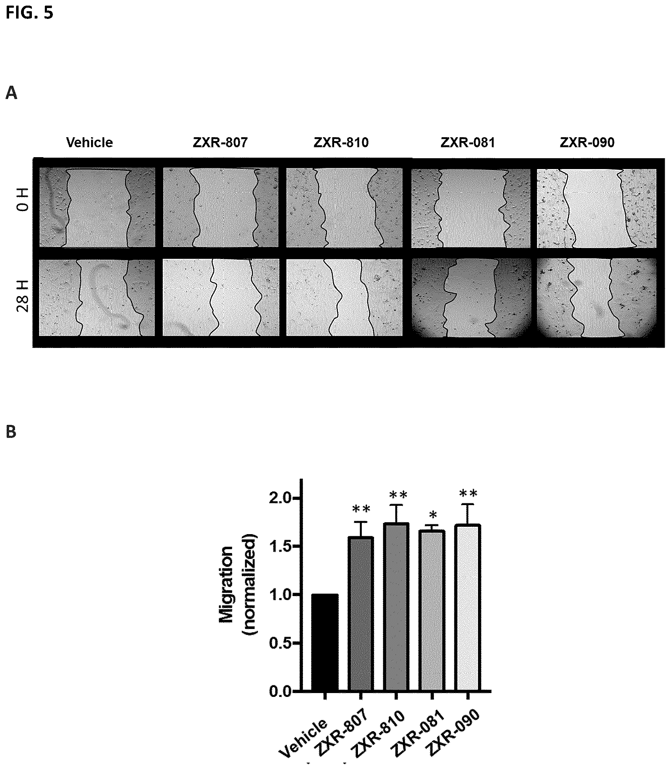

Fig. 5. Tob1 inhibitors promote cell migration in H9C2 cells. (A) Representative images of the migration of H9C2 cells following treatment with the lead Tob1 inhibitors at 100 nM. (B) Summary graph showing a higher rate of migration with the inhibitors compared to the control, vehicle-treated cells. Shown are normalized data means ± SEM. Asterisks indicate ** P<0,01 according to unpaired two-tailed Student's t-test.
Fig. 6. Tob1 inhibition in hiPSC cardiomyocytes promotes proliferation. Proliferation rate is assessed by measuring the quantity of cells metabolically active (producing ATP) with CellTiterGlo at 144 hours (6 days) (A) and 336 hours after (14 days) after treatment with 8 nM of Tob1 inhibitors (B) nuclear Ki67 signal intensity close to the scratch (wound) area and away from the scratch (wound) area in hiPSC-CMs treated with 8 nM Tob1 inhibitors for 144 hrs. Shown are normalized data means ± SEM. Asterisks indicate *** P<0,001; ** P<0,01 and *P<0,05 according to unpaired two-tailed Student's t-test for proliferation and one-way ANOVA followed by Dunnett's multiple comparison test for Ki67 nuclear intensity levels..
Fig. 7: Tob1 inhibition inhibits hiPSC cardiomyocyte differentiation. cTnT signal intensity was measured in a migration assay, of hiPSC-CMs, in the region close to the wound, following treatment with 8 nM Tob1 inhibitors for 144 hours. Shown are normalized data means ± SEM. Asterisks indicate *** P<0,001; ** P<0,01 and *P<0,05 according to one-way ANOVA followed by Dunnett's multiple comparison test.

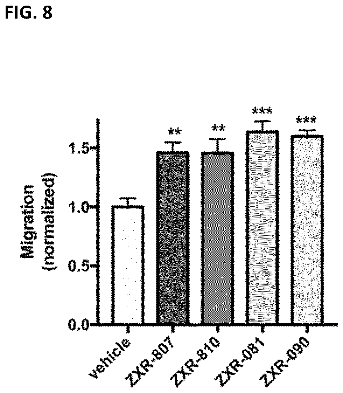

Fig. 8. Tob1 inhibitors promote cell migration in hiPSC cardiomyocytes. 144 hours after treatment of hiPSC cardiomyocytes with 8nM ZXR-807, ZXR-810, ZXR-081 and ZXR-090 migration rate was evaluated in cells treated with the Tob1 inhibitors and normalized to vehicle treated cells. Shown are normalized data means ± SEM. Asterisks indicate *** P<0,001; ** P<0,01 according to one-way ANOVA followed by Dunnett's multiple comparison test.

### DETAILED DESCRIPTION

Terms not specifically defined herein should be given the meanings that would be given to them by one of skill in the art in light of the disclosure and the context. As used in the specification, however, unless specified to the contrary, the following terms have the meaning indicated, and the following conventions are adhered to.

Throughout the present specification and the accompanying clauses, the words "comprise" and variations such as "comprises", "comprising" are to be interpreted inclusively. That is, these words are intended to convey the possible inclusion of other elements or integers not specifically recited, where the context allows. The word "comprise" also includes the term "consists of".

For the purposes of the present invention, any ranges given include both the lower and the upper end-points of the range.

In a first aspect the present invention provides compounds of formula (I), stereoisomer, solvate or acceptable salts thereof.

In the context of the invention, the term "alkyl" refers to a straight or branched hydrocarbon chain radical containing no unsaturation, and which is attached to the rest of the molecule by a single bond. Typical alkyl groups have from 1 to about 10, 1 to about 8, or 1 to about 6 carbon atoms, e. g., methyl, ethyl, n-propyl, i-propyl, n-butyl, t-butyl, n-pentyl, etc.

In the context of the invention, the term "alkenyl" refers to a straight or branched hydrocarbon chain radical containing at least two carbon atoms and at least one C=C double bond, and which is attached to the rest of the molecule by a single bond. Typical alkenyl radicals have from 2 to about 10, 2 to about 8 or 2 to about 6 carbon atoms. In a particular embodiment, the alkenyl group is vinyl, 1-methyl-ethenyl, 1-propenyl, 2-propenyl, or butenyl.

In the context of the invention, the term "alkynyl" refers to a straight or branched hydrocarbon chain radical containing one or more C=C triple bonds. Examples of alkynyl groups include, but are not limited to, ethynyl, 2-propynyl, and 2-butynyl.

In the context of the invention, the term "halogen" refers to bromo, chloro, iodo or fluoro.

In the context of the invention, the term "haloalkyl" refers to a straight or branched hydrocarbon chain radical containing no unsaturation, wherein one or more of the hydrogen atoms are replaced by halogen. Illustrative non-limitative examples of haloalkyl are chloromethyl, trifluoromethyl, 1-chloro-2-fluoroethyl, and the like.

In one embodiment of the first aspect of the invention, R₂ₐ to R₂ₑ are the same or different and represent hydrogen or halogen. In another embodiment of the first aspect of the invention, one of R₂ₐ to R₂ₑ is halogen and the others are hydrogen.

In another embodiment of the first aspect of the invention R₁ represents -H or C(O)R₆. In another embodiment of the first aspect of the invention, R₆ represents H; (C₁-C₁₀)alkyl; or (C₁-C₁₀)alkyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; particularly, R₆ represents H or (C₁-C₁₀)alkyl.

In another embodiment of the first aspect of the invention, R₃ and R₄ are the same or different and represent (C₁-C₁₀)alkyl; or (C₁-C₁₀)alkyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; particularly, R₆ represents H or (C₁-C₁₀)alkyl.

In an alternative embodiment of the first aspect of the invention, R₃ and R4 form, together with the N heteroatom to which they are attached, a ring system consisting of one ring. In one embodiment, the ring system consists of one saturated 5- or 6-membered ring, particularly a saturated 6-membered ring. In another embodiment, the ring system consists of a saturated 6-membered ring, wherein one of the members is the N atom to which R₃ and R₄ are attached, another member is O or S, and the remaining members represent C(Rₓ)₂, being Rₓ as defined above. In another embodiment, the ring system consists of a saturated 6-membered ring wherein one of the members is the N atom to which R₃ and R₄ are attached, another member is O or S, and the remaining members represent CH₂.

In another embodiment, the compound of formula (I) is selected from the group consisting of:
rel-[(1R,2R,4R)-4-amino-2-(4-chlorophenyl)cyclopentyl]-thiomorpholino- methanone;
rel(1R,2R,4R)-4-amino-2-(4-chlorophenyl)-N-isopropyl-N-methyl- cyclopentanecarboxamide;
rel-[(1R,2R,4R)-4-amino-2-(4-chlorophenyl)cyclopentyl]-morpholino-methanone hydrochloride;
rel-N-[(1R,3R,4R)-3-(4-chlorophenyl)-4-(morpholine-4-carbonyl)cyclopentyl] acetamide;
and any solvate, salt or stereoisomer thereof.

In the context of the present invention, the term "salt" must be understood as any form of a compound used in accordance with this invention in which said compound is in ionic form or is charged and coupled to a counter-ion (a cation or anion) or is in solution. This definition also includes quaternary ammonium salts and complexes of the active molecule with other molecules and ions, particularly, complexes formed via ionic interactions. This definition includes in particular physiologically acceptable salts.

In the context of the present invention, the term "pharmaceutically acceptable salts" means any salt that is tolerated physiologically (normally meaning that it is not toxic, particularly, as a result of the counter-ion) when used in an appropriate manner for a treatment, applied or used, particularly, in humans and/or mammals. These physiologically acceptable salts may be formed with cations or bases and, in the context of this invention, are understood to be salts formed by at least one compound used in accordance with the invention -normally an acid (deprotonated)- such as an anion and at least one physiologically tolerated cation, preferably inorganic, particularly when used in humans and/or mammals. Salts with alkali and alkali earth metals are preferred particularly, as well as those formed with ammonium cations (NH4+). Preferred salts are those formed with (mono) or (di)sodium, (mono) or (di)potassium, magnesium, or calcium. These physiologically acceptable salts may also be formed with anions or acids and, in the context of this invention, are understood as being salts formed by at least one compound used in accordance with the invention - normally protonated, for example in nitrogen - such as a cation and at least one physiologically tolerated anion, particularly when used on humans and/or mammals. This definition specifically includes in the context of this invention a salt formed by a physiologically tolerated acid, i.e., salts of a specific active compound with physiologically tolerated organic or inorganic acids - particularly when used on humans and/or mammals. Examples of this type of salts are those formed with hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid or citric acid.

In the context of the invention, a "cosmetically acceptable salt" refers to a salt prepared from a cosmetically acceptable base, such as an inorganic base and an organic base, or a salt prepared from a cosmetically acceptable acid. Representative salts derived from inorganic bases include aluminum, ammonium, calcium, copper, ferric, ferrous, lithium, magnesium, manganic, manganous, ammonium, potassium, sodium, and zinc salts. Representative salts derived from cosmetically acceptable organic bases include salts of primary, secondary and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, and trimethamine.

In the context of the present invention, the term "solvate" should be understood as meaning any form of a compound in accordance with the invention in which the said compound is bonded by a noncovalent bond to another molecule (normally a polar solvent), including especially hydrates and alcoholates, such as, for example, methanolate. A preferred solvate is the hydrate.

Any compound of formula (I) referred to herein is intended to represent such a specific compound, as well as certain variations or forms. In particular, compounds referred to herein may have asymmetric centres and therefore exist in different enantiomeric or diastereomeric forms. Thus, any compound of formula (I) referred to herein is intended to represent any one of a racemate, one or more enantiomeric forms, one or more diastereomeric forms, and mixtures thereof. Likewise, stereoisomerism or geometric isomerism about the double bond is also possible, therefore in some cases the molecule could exist as (E)-isomer or (Z)-isomer (trans and cis isomers). If the molecule contains several double bonds, each double bond will have its own stereoisomerism, which could be the same as or different from, the stereoisomerism of the other double bonds of the molecule. Furthermore, compounds referred to herein may exist as atropoisomers. All the stereoisomers including enantiomers, diastereoisomers, geometric isomers and atropoisomers of the compounds referred to herein, and mixtures thereof, are considered within the scope of the present invention.

Furthermore, any compound of formula (I) referred to herein may exist as tautomer. Specifically, the term tautomer refers to one of two or more structural isomers of a compound that exist in equilibrium and are readily converted from one isomeric form to another.

The compounds of the invention can be prepared following any of the well-known protocols known to those skilled in the art. Illustrative non-limitative examples are provided below.

In one embodiment, the compounds of the invention are obtained from the compound of formula (II): 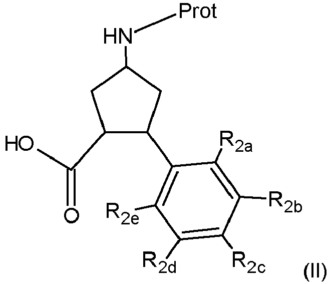 wherein R₂ₐ to R₂ₑ are as defined above, and "Prot" represents a N-protecting group. Illustrative non-limitative examples of N-protecting groups are those which can be used for the synthesis of (oligo)peptides. Such groups are known to the person skilled in the art. Examples of suitable N-protecting groups include carbonyl type protective groups, for instance 'Z' (benzyloxycarbonyl), 'Boc' (i.e. t-butyloxycarbonyl), 'For' (i.e. formyl) and `PhAc' (phenacetyl), and FMOC (9-fluorenylmethoxycarbonyl). The groups For or PhAc may be introduced and cleaved enzymatically using the enzymes Peptide Deformylase or PenG acylase, respectively. Chemical cleavage methods are generally known in the art.

The particular reaction conditions to which the compound of formula (II) is subjected, will depend on the meaning of R₃ and R₄, mainly of whether they are, or aren't, forming a heterocycle together with the N atom to which they are attached.

Particular conditions for each one of the embodiments are provided below. But, mainly, when R₃ and R₄ form together with the N atom to which they are attached a heterocycle further including a -O- heteroatom, the compound of formula (II) is mixed with diisopropylethylamine and propanephosphonic acid anhydride; and when R₃ and R₄ form together with the N atom to which they are attached a heterocycle further including a -S- heteroatom, the compound of formula (II) is mixed with 1-hydroxybenzotriazole, a carbodiimide (such as 1-ethyl-3-(3-dimethylaminopropyl)carbodiimide hydrochloride), diisopropylethylamine and thiomorpholine. Once formed the heterocycle, the resulting compound is subjected to a deprotecting step. The conditions of deprotection are well-known to those skilled in the art, for instance, acidic medium.

Alternatively, when R₃ and R₄ are not forming a heterocycle with the N atom, the compound of formula (II) reacts with an amine of formula (III):

HNR₃R₄ (III)

wherein R₃ and R₄ are as defined above. Once the reaction has been completed, the deprotection step is carried out following any of the well-known protocols.

In a further aspect the present invention provides pharmaceutical compositions comprising at least one or more compounds of formula (I), as well as pharmaceutically acceptable excipients and/or carriers.

Pharmaceutically acceptable adjuvants, vehicles or excipients which may be used in such compositions are adjuvants, vehicles or excipients known to those skilled in the art or commonly used in the preparation of therapeutic compositions, which may be selected, for example, from the group consisting of excipients, fillers, solvents, diluents, surfactants, colorants, preservatives, disintegrants, sliding agents, lubricants, flavoring agents or binders.

The term "pharmaceutically acceptable" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans without excessive toxicity, irritation, allergic response, immunogenicity or other problems or complications commensurate with a reasonable benefit/risk ratio in animals and, particularly, in humans. The selection of physiologically compatible adjuvants or the amount of adjuvants to be used depends on the form of administration of the pharmaceutical composition, i.e., oral, subcutaneous, parenteral, intravenous, intraperitoneal, intradermal, intramuscular, intranasal, buccal, rectal, otic or intratympanic. Preparations in the form of tablets, dragees, capsules, granules, pills, drops, in particular otic drops, juices or syrups are preferably suitable for oral administration; solutions, suspensions, easily reconstitutable dry preparations or also sprays are preferably suitable for parenteral, topical or inhalation administration.

For instance, for oral administration in the form of a tablet or capsule, the active drug components can be combined with an oral, non-toxic, pharmaceutically acceptable, inert carrier such as lactose, starch, sucrose, glucose, methyl cellulose, magnesium stearate, dicalcium phosphate, calcium sulphate, mannitol, sorbitol and the like; for oral administration in liquid form, the oral drug components can be combined with any oral, non-toxic, pharmaceutically acceptable inert carrier such as ethanol, glycerol, water, and the like. Moreover, when desired or necessary, suitable binders, lubricants, disintegrating agents, and colouring agents can also be incorporated into the mixture. Suitable binders include starch, gelatine, natural sugars such as glucose or beta-lactose, corn sweeteners, natural and synthetic gums such as acacia, tragacanth, or sodium alginate, carboxymethylcellulose, polyethylene glycol, waxes, and the like. Lubricants used in these dosage forms include sodium oleate, sodium stearate, magnesium stearate, and the like. Disintegrators include, without limitation, starch, methyl cellulose, agar, bentonite, xanthan gum, and the like.

Gelatine capsules contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain colouring and flavouring to increase patient acceptance.

The combinations of this invention may be formulated such that, although the active ingredients are combined in a single dosage unit, the physical contact between the active ingredients is minimized. In order to minimize contact, for example, one or more of the active ingredients may be enteric coated. By enteric coating one of the active ingredients, it is possible not only to minimize the contact between the combined active ingredients, but also, it is possible to control the release of one of these components in the gastrointestinal tract such that one of these components is not released in the stomach but rather is released in the intestines. Another embodiment of this invention provides for a combination product wherein one or more of the active ingredients is coated with a sustained-release material which effects a sustained-release throughout the gastrointestinal tract and also serves to minimize physical contact between the combined active ingredients. Furthermore, the sustained-released component can be additionally enteric coated such that the release of this component occurs only in the intestine. Still another approach would involve the formulation of a combination product in which the one or more components is coated with a sustained and/or enteric release polymer, and the other(s) component is also coated with a polymer such as a low viscosity grade of hydroxypropyl methylcellulose or other appropriate materials as known in the art, in order to further separate the active components. The polymer coating serves to form an additional barrier to interaction with the other component.

Dosage forms of the combination products of the present invention wherein one active ingredient is enteric coated can be in the form of tablets such that the enteric coated component and the other active ingredients are blended together and then compressed into a tablet or such that the enteric coated component is compressed into one tablet layer and the other active ingredient is compressed into an additional layer. Optionally, in order to further separate the two layers, one or more placebo layers may be present such that the placebo layer is between the layers of active ingredients. In addition, dosage forms of the present invention can be in the form of capsules wherein one active ingredient is compressed into a tablet or in the form of a plurality of microtablets, particles, granules or non-perils, which are then enteric coated. These enteric coated microtablets, particles, granules or non-perils are then placed into a capsule or compressed into a capsule along with a granulation of the other active ingredient.

By "therapeutically effective amount", it is understood the amount of the compound or composition that, when administered, is sufficient to prevent development of, or alleviate to some extent, one or more of the symptoms of the disease which is addressed.

The dosage administered of the pharmaceutical composition will, of course, vary depending on the use and known factors such as the age, health, and weight of the recipient; nature and extent of symptoms, concurrent treatments, if any, frequency of treatment, and the effect desired. The recipient may be any type of mammal, but is preferably a human.

In a further aspect the present invention provides topical cosmetic compositions comprising at least one compound of formula (I), as well as cosmetically acceptable excipients and/or carriers.

The cosmetic compositions of the invention can include diluents, excipients, solubilizing agents, emulsifying agents, and salts known to be useful for cosmetic compositions. Examples of suitable agents include thickeners, buffers, preservatives, surface active agents, neutral or cationic lipids, lipid complexes, liposomes, and penetration enhancers. In certain embodiments, the cosmetic compositions further include other cosmetic ingredients known in the art.

In certain embodiments, the cosmetic composition can include one or more penetration enhancers. Numerous types of penetration enhancers are known, such as fatty acids, bile salts, chelating agents, surfactants and non-surfactants. Fatty acids and their derivatives which act as penetration enhancers include, for example, cabrylic acid, oleic acid, lauric acid, capric acid, caprylic acid, hexanoic acid, myristic acid, palmitic acid, valeric acid, stearic acid, linoleic acid, linolenic acid, arachidonic acid, oleic acid, elaidic acid, erucic acid, nervonic acid, dicaprate, tricaprate, recinleate, monoolein (also known as 1-monooleoyl-rac-glycerol), dilaurin, arachidonic acid, glyceryll-monocaprate, 1-dodecylazacycloheptan-2-one, acylcarnitines, acylcholines, mono- and di-glycerides and physiologically acceptable salts thereof (e.g., oleate, laurate, caprate, myristate, palmitate, stearate, linoleate).

In certain embodiments, the cosmetic composition further includes other cosmetic ingredients known in the art to be useful for cosmetic, skincare, and/or dermatological applications (e.g., anti-wrinkle active ingredients including flavone glycosides such as alpha-glycosylrutin; coenzyme Q10; vitamin E and derivatives; as well as sunblock ingredients, moisturizers, and perfumes).

The cosmetic compositions of the invention are administered for "cosmetic" or "skincare" (e.g., dermatologic) applications, either alone or as an "additive" in combination with other suitable agents or ingredients. As used herein, "cosmetic" and "skincare" applications includes, for example, preventive and/or restorative applications in connection with dermatological changes in the skin, such as, for example, during pre-mature skin aging; dryness; roughness; formation of dryness wrinkles; itching; reduced re-fatting (e.g., after washing); visible vascular dilations (e.g., telangiectases, cuperosis); flaccidity; formation of wrinkles and lines; local hyperpigmentation; hypopigmentation; incorrect pigmentation (e.g., age spots); increased susceptibility to mechanical stress (e.g., cracking); skin-sagging (e.g., lack of firmness) and the appearance of dry or rough skin surface features.

The cosmetic compositions of the invention are formulated for topical administration. Such compositions can be administered topically in any of a variety of forms. Such compositions are suitable in the context of the use described herein for application to the skin.

Compositions for topical administration include dermal patches, ointments, lotions, serums, creams, gels, hydrogels, pastes, foams, oils, semi-solids, shampoos, soaps, drops, sprays, films, liquids, and powders.

In further aspects the present invention provides the use of the compounds of formula (I), stereoisomers, solvates or salts thereof, in the repair of tissues and regeneration of organs.

In the context of the invention, "regeneration" means reconstruction and reproduction of lost tissues, destroyed tissues and damaged tissues to an initial state recovering its function.

In the context of the invention, "repair" means healing of a wounded tissue as in case wherein the structure and functions of the wound have not yet been fully restored.

The beneficial effects of the compounds and compositions of the invention need not only be on directly damaged or injured cells. In some embodiments of the invention, for example, the cells of the damaged tissue that are influenced by the disclosed methods are healthy cells. However, in several embodiments, the cells of the damaged tissue that are influenced by the disclosed methods are damaged cells.

In the context of the invention, the therapeutic uses are related to damaged cells resulting from a pathological condition or event. Whereas the cosmetic (non-therapeutic) uses provided in the context of the invention, are related to the external appearance of the subject (i.e., the tissue injury or damage is not due to a pathological condition or event).

In several embodiments, regeneration comprises improving the function of the tissue. For example, in certain embodiments in which cardiac tissue is damaged, functional improvement may comprise increased cardiac output, contractility, ventricular function and/or reduction in arrhythmia (among other functional improvements). For other tissues, improved function may be realized as well, such as enhanced cognition in response to treatment of neural damage, improved blood-oxygen transfer in response to treatment of lung damage, improved immune function in response to treatment of damaged immunological-related tissues.

The repair of the damaged tissue may comprise both anatomical repair (e.g., tissue regeneration) and functional repair.

In several embodiments, the damaged tissue is in need of repair, regeneration, or improved function due to an acute event. Acute events include, but are not limited to, trauma such as laceration, crush or impact injury, shock, loss of blood or oxygen flow, infection, chemical or heat exposure, poison or venom exposure, drug overuse or overexposure, and the like. For example, in several embodiments, the damaged tissue is cardiac tissue and the acute event comprises a myocardial infarction. In additional embodiments, the tissue is damaged due to chronic disease or ongoing injury. For example, progressive degenerative diseases can lead to tissue damage that propagates over time (at times, even in view of attempted therapy). Chronic disease need not be degenerative to continue to generate damaged tissue, however. Cardiac tissue, in several embodiments, is also subject to damage due to chronic disease, such as for example congestive heart failure, ischemic heart disease, diabetes, valvular heart disease, dilated cardiomyopathy, infection, among others. Other sources of damage also include, but are not limited to, injury, age-related degeneration, cancer treatment, and infection. In several embodiments, the regenerative cells are from the same tissue type as is in need of repair or regeneration. In several other embodiments, the regenerative cells are from a tissue type other than the tissue in need of repair or regeneration. In several embodiments, the regenerative cells comprise somatic cells, while in additional embodiments, they comprise germ cells.

In a further aspect the present invention refers to the use of TOB1 inhibitors in the repair of a tissue or regeneration of an organ.

TOB1 encodes a member of the tob/btg1 family of anti-proliferative proteins. Uniprot accession numbers of the human and murine protein sequences are, respectively, P50616 (last updated 1996-10-01 v1) and Q61471 (2013-09-18 v2). Genbank accession numbers of the human and murine nucleotide sequences are, respectively, Human Gene ID: 10140 and mouse Gene ID: 22057.

In the context of the present invention, the term "TOB1 inhibitor" refers to a compound with the ability of reducing or avoiding TOB1 activity. There are well-known protocols in the state of the art to determine TOB1 activity (Doidge et al., 2012) It is considered that a compound inhibits TOB1 activity when cellular proliferation increases with statistical significance above control levels as TOB1 was shown to be an anti-proliferative protein (Winkler et al., 2010) . There are well-known small fragments inhibitors of TOB1 in the state of the art (Bai et al., 2015). Based on the predicted structure, it can be determined or confirmed whether a compound is a TOB1 inhibitor or not.

To those skilled in the art, other objects, advantages or features of the invention will be apparent in part from the description or in part from the practice of the invention. The following examples are provided by way of illustration or are not intended to be limiting of the present invention.

### EXAMPLES

### MATERIALS AND METHODS

NMR measurements were performed on Bruker NMR machine 300 MHz. UHPLC measurements were performed on instrument uHPLC Agilent 1290 Infinity, column: ACQUITY UHPLC BEH C18 (1.7µm) 2.1 mmx50mm, temperature 40 °C, detection: DAD - 6120 Quadrupole; Solvent A: Water+0.1% formic acid, Solvent B: MeCN+0.1% formic acid; Gradient conditions A: 0 min. 2% B; 0.5 min. 2% B; 3.0 min. 98% B; 3.5 min. 98% B; 3.6 min. 2% B, flow 0.6 mL/min

Gradient conditions B: 0min. 2%B, 0.2min. 2%B, 3.9min. 98%B, 4.1min. 98%B, 4.11min. 2%B, 4.5min. 2%B, flow 1.0 mL/min; 200-300nm.

### Chemical synthesis

### Example 1: Synthesis of rel-N-[(1 R,3R,4R)-3-(4-chlorophenyl)-4-(morpholine-4-carbonyl) cyclopentyl]acetamide (ZXR-090):

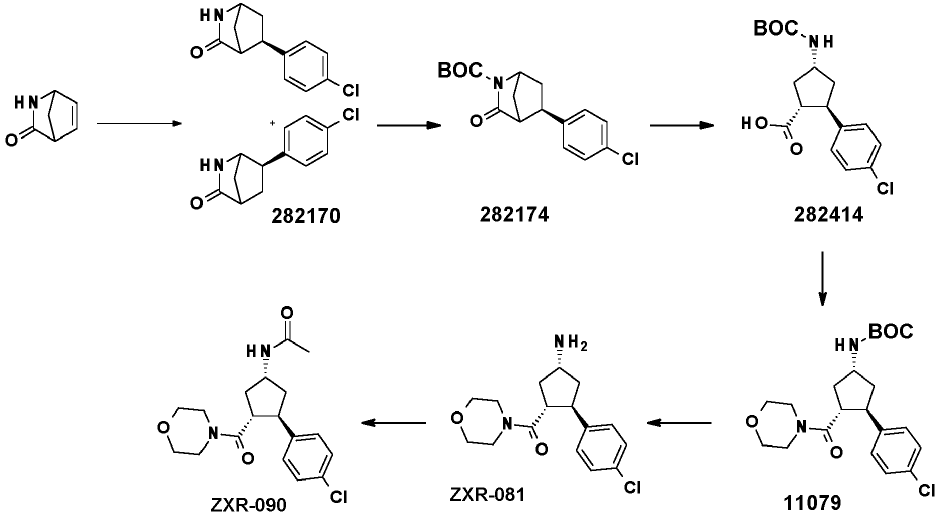

### a) Preparation of compound 282170, rel-(5R)-5-(4-chlorophenyl)-2-azabicyclo[2.2.1]heptan-3-one:

To a solution of 2-azabicyclo[2.2.2]hept-5-en-3-one (45.0 g, 412 mmol, 1 equiv.) in dry tetrahydrofuran (700 mL) under nitrogen atmosphere was added 1-chloro-4-iodobenzene (100 g, 412 mmol, 1 equiv.), triethylamine (115 mL, 824 mmol, 2 equiv.), formic acid (22.5 mL, 598 mmol, 1.45 equiv.) and bis(triphenylphosphine)palladium chloride (12 g, 17.5 mmol, 0.05 equiv.) and the reaction mixture was heated to reflux.

The reaction progress was monitored by UHPLC and full conversion was observed after 3 hours.

The reaction mixture was concentrated in vacuo. The residue was diluted with ethyl acetate (1000 mL) and 1N aqueous HCl (100 mL). Both layers were separated and the organic layer was filtered through celite and washed with brine (3x300 mL). The organic phase was dried over MgSO₄ and then concentrated in vacuo.

The crude was suspended in a mixture of petroleum ether/dichloromethane (900 mL, 8:1). The solid was filtered, washed with cold petroleum ether/dichloromethane (100 mL, 8:1) and dried in vacuo, providing the title compound as mixture of two regioisomers in ratio 1:1 (70.8 g, 77%).

MS (ES-APCI) m/z: [M+H]⁺ calc. for C₁₂H₁₃ClNO ⁺222.1, found 222.2.

1H NMR (300 MHz, CDCl₃, d in ppm): 7.23-7.19 (two overlapping d, 4H), 7.12-7.07 (two overlapping d, 4H), 6.94 (bs, 1H), 6.81 (bs, 1H), 3.97-3.93 (m, 1H), 3.81-3.77 (m, 1H), 3.22-3.14 (m, 2H), 2.76-2.72 (m, 2H), 2.23-2.05 (m, 2H), 1.97-1.86 (m, 4H), 1.65 (t, 2H, J=9.7 Hz).

### b) Preparation of compound 282174, tert-butyl rel-(5R)-5-(4-chlorophenyl)-3-oxo-2-azabicyclo[2.2.1]heptane-2-carboxylate:

To a solution of rel-(5R)-5-(4-chlorophenyl)-2-azabicyclo[2.2.1]heptan-3-one (as a mixture of two regioisomers, 70.8 g, 319 mmol, 1 equiv.) in dichloromethane (1000 mL) was added di-tert-butyl dicarbonate (139.5 g, 638 mmol, 2 equiv.), DMAP (39.0 g, 319 mmol, 1 equiv.) and triethylamine (110 mL, 798 mmol, 2.5 equiv.) and the reaction mixture was allowed to stir at room temperature (note: During the first hour, violent evolution of carbon dioxide was observed).

The reaction progress was monitored by UHPLC and full conversion was observed after 12 hours.

The reaction mixture was concentrated in vacuo. The residue was dissolved in methyl tert-butyl ether (1000 mL) and was washed with aqueous citric acid (5 wt%, 300 mL), saturated sodium hydrogen carbonate (200 mL) and brine (200 mL). The organic phase was dried over MgSO₄ and then concentrated in vacuo. To separate the two regioisomer, the crude was purified by flash column chromatography (cyclohexane/methyl tert butyl ether) to get the target intermediate (41.4 g, 40%).

MS (ES-APCI) m/z: [M-Boc+H]⁺ calc. for C₁₂H₁₃ClNO⁺222.6, found 222.2.

1H NMR (300 MHz, CDCl₃, d in ppm): 7.22 (d, 2H, J=8.5 Hz), 7.09 (d, 2H, J=8.5 Hz), 4.58-4.56 (m, 1H), 3.31 (dd, 1H, J=3.3, 8.8 Hz), 2.87 (d, 1H, J=2.9 Hz), 2.36-2.27 (m, 1H), 1.96 (dd, 1H, J=2.5, 5.2 Hz), 1.93-1.89 (m, 1H), 1.70-1.67 (m, 1H), 1.47 (s, 9H).

### c) Preparation of compound 282414, rel-(1R,2R,4R)-4-(tert-butoxycarbonylamino)-2-(4-chlorophenyl)cyclopentanecarboxylic acid:

To a solution of tert-butyl rel-(5R)-5-(4-chlorophenyl)-3-oxo-2-azabicyclo[2.2.1]heptane-2-carboxylate (40.0 g, 124 mmol, 1 equiv.) in tetrahydrofuran (750 mL) at 0 °C was added a solution of lithium hydroxide (7.4 g, 310 mmol, 2.5 equiv.) in water (170 mL) and the reaction mixture was allowed to stir at this temperature.

The reaction progress was monitored by UHPLC and full conversion was observed after 3 hours.

The reaction mixture was diluted with water (1000 mL) and was washed with ethyl acetate (600 mL) to remove any organic impurities. The water phase was acidified with aqueous HCl (1N, 70 mL) until pH-5-6. It was then extracted with ethyl acetate (2x800 mL), the organic extract was dried over MgSO₄ and then concentrated in vacuo. The product was used without further purification in the next step (36.5 g, 86%).

MS (ES-APCI) m/z: [M-H]⁻ calc. for C₁₇H₂₁ClNO₄⁻ 338.1, found 338. 2.

1H NMR (300 MHz, CDCl₃, d in ppm): 7.20 (signals overlapping with CDCl₃ signal, 4H), 3.99-3.97 (m, 1H), 3.62-3.56 (m, 1H), 3.45-3.36 (m, 1H), 2.56-2.50 (m, 1H), 2.19-2.16 (m, 1H), 1.89-1.81 (m, 2H), 1.73-1.58 (m, 1H), 1.37 (s, 9H).

### d) Preparation of compound 11079, tert-butyl rel-N-[(1R,3R,4R)-3-(4-chlorophenyl)-4-(morpholine-4-carbonyl)cyclopentyl]carbamate:

To a solution of rel-(1R,2R,4R)-4-(tert-butoxycarbonylamino)-2-(4-chlorophenyl)cyclopentanecarboxylic acid (500 mg, 1.47 mmol, 1 equiv.) in ethyl acetate (5 mL) was added diisopropylethylamine (0.5 mL, 2.94 mmol, 2 equiv.) and propanephosphonic acid anhydride (-50 wt% in ethyl acetate, 1 mL, 1.61 mmol, 1.1 equiv.). After all solids were dissolved and the reaction mixture became clear solution, morpholine (0.14 mL, 1.61 mmol, 1.1 equiv.) was added and the reaction mixture was allowed to stir at room temperature.

The reaction progress was monitored by UHPLC and full conversion was observed after 30 min.

The reaction mixture was diluted with ethyl acetate (15 mL) and was washed with aqueous sodium hydrogen carbonate (20 mL), water (20 mL) and brine (20 mL). The organic phase was dried over MgSO₄ and then concentrated in vacuo. The product was used without further purification in the next step (505 mg, 84%).

MS (ES-APCI) m/z: [M-Boc+H]⁺ calc. for C16H22ClN2O2⁺ 309.2, found 309.2.

1H NMR (300 MHz, CDCl₃, d in ppm): 7.27 (d, 2H, J=8.4 Hz), 7.16 (d, 2H, J=8.4 Hz), 5.80-5.77 (m, 1H), 4.31-4.29 (m, 1H), 3.77-3.64 (m, 2H), 3.53-3.41 (m, 4H), 3.18-2.99 (m, 4H), 2.39-2.29 (m, 1H), 2.19-2.15 (m, 1H), 1.98-1.89 (m, 2H), 1.44 (s, 9H).

### e) Preparation of compound ZXR-081, rel-[(1R,2R,4R)-4-amino-2-(4-chlorophenyl)cyclopentyl]-morpholino-methanone hydrochloride:

To a solution of tert-butyl rel-N-[(1R,3R,4R)-3-(4-chlorophenyl)-4-(morpholine-4-carbonyl)cyclopentyl]carbamate (580 mg, 1.23 mmol, 1 equiv.) in dichloromethane (5 mL) was added HCl (4N in dioxane, 3 mL, 12.00 mmol, 10 equiv.) and the reaction mixture was allowed to stir at room temperature.

The reaction progress was monitored by UHPLC (acidic buffer, gradient conditions B) and full conversion was observed after 20 hours.

The reaction mixture was concentrated in vacuo, after which the crude was dissolved in acetonitrile/water (1:1) and freeze-dried. The product as HCl salt was used without further purification in the next step (430 mg, 99%).

100 mg sample was purified by preparative HPLC (Column: XBridge BEH Prep C18 5 µm, 19 mm X 150 mm; Solvent system acetonitrile/Water+ 5mM NH₄HCO₃) to afford the product with high purity for bio assays (37 mg).

MS (ES-APCI) m/z: [M+H]⁺ calc. for C₁₆H₂₂ClN₂O₂⁺ 309.1, found 309.2.

1H NMR (300 MHz, MeOD-d₄, δ in ppm): 7.37-7.30 (overlapping peaks, 4H), 3.98-3.93 (m, 1H), 3.73-3.33 (m, 8H), 3.24-3.18 (m, 1H), 3.06-2.98 (m, 1H), 2.58-2.49 (m, 1H), 2.39-2.20 (m, 2H), 2.07-1.98 (m, 1H).

### f) Preparation of compound ZXR-090, rel-N-[(1R,3R,4R)-3-(4-chlorophenyl)-4-(morpholine-4-carbonyl)cyclopentyl]acetamide:

To a solution of [(1R,2R,4R)-4-amino-2-(4-chlorophenyl)cyclopentyl]-morpholino-methanone hydrochloride (50 mg, 0.14 mmol, 1 equiv.) in dichloromethane (3 mL) was added diisopropyethylamine (0.1 mL, 0.58 mmol, 4 equiv.) and acetyl chloride (0.013 mL, 0.16 mmol, 1.1 equiv.) and the reaction mixture was allowed to stir at room temperature.

The reaction progress was monitored by UHPLC (acidic buffer, gradient conditions B) and full conversion was observed after 20 hours.

The reaction mixture was concentrated in vacuo and the crude material was purified by preparative HPLC (Column: XBridge BEH Prep C18 5 µm, 19 mm X 150 mm; Solvent system acetonitrile/Water+0.1% formic acid) to afford the title compound (34.8 mg, 68%).

MS (ES-APCI) m/z: [M+H]⁺ calc. for C₁₈H₂₄ClN₂O₃⁺ 351.1, found 351.2.

1H NMR (300 MHz, CDCl₃, δ in ppm): 7.33 (bs, 1H), 7.23 (d, 2H, J=8.5 Hz), 7.10 (d, 2H, J=8.5 Hz), 4.50-4.46 (m, 1H), 3.77-3.60 (m, 2H), 3.49 (dt, 1H, J=2.8, 7.9 Hz), 3.40-3.23 (m, 3H), 3.15-3.02 (m, 3H), 2.95-2.89 (m, 1H), 2.30-2.177 (m, 2H), 1.96 (s, 3H), 1.92-1.82 (m, 2H).

### Example 2: Synthesis of compound ZXR-807, rel-[(1R,2R,4R)-4-amino-2-(4-chlorophenyl)cyclopentyl]-thiomorpholino-methanone:

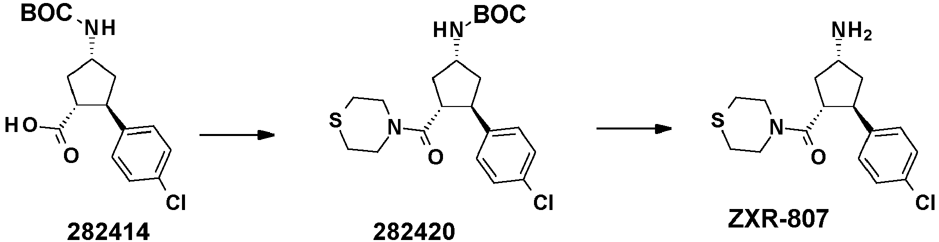

### a) Preparation of compound 282420, tert-butyl rel-N-[(1R,3R,4R)-3-(4-chlorophenyl)-4-(thiomorpholine-4-carbonyl)cyclopentyl]carbamate:

To a solution of rel-(1R,2R,4R)-4-(tert-butoxycarbonylamino)-2-(4-chlorophenyl)cyclopentanecarboxylic acid (7.0 g, 20.6 mmol, 1.0 equiv.) in dichloromethane (100 mL) was added 1-hydroxybenzotriazole (4.4 g, 30.9 mmol, 1.5 equiv.), 1-Ethyl-3-(3-dimethylaminopropyl)carbodiimide hydrochloride (8.0 g, 41.2 mmol, 2.0 equiv.), diisopropylethylamine (14.4 mL, 82.4 mmol, 4.0 equiv.) and thiomorpholine (4.2 mL, 41.2 mmol, 2.0 equiv.) was added and the reaction mixture was allowed to stir at room temperature.

The reaction progress was monitored by UHPLC and full conversion was observed after 20 h.

The reaction mixture was washed with citric acid (5 wt%, 2x100 mL), ammonium chloride (200 mL) and sodium hydrogencarbonate (200 mL). The organic phase was dried over MgSO₄ and then concentrated in vacuo. The product was used without further purification in the next step (7.2 g, 83%).

MS (ES-APCI) m/z: [M-Boc+H]⁺ calc. for C₁₆H₂₂ClN₂OS⁺ 325.1, found 325.1.

1H NMR (300 MHz, CDCl₃, d in ppm): 7.21 (d, 2H, J=8.4 Hz), 7.09 (d, 2H, J=8.4 Hz), 5.73-5.71 (m, 1H), 4.30-4.19 (m, 1H), 3.92-3.84 (m, 1H), 3.73-3.65 (m, 1H), 3.46-3.34 (m, 3H), 3.05-2.96 (m, 1H), 2.53-2.41 (m, 2H), 2.39-2.20 (m, 2H), 2.14-1.78 (m, 4H), 1.38 (s, 9H).

b) Preparation of compound ZXR-807, rel-[(1R,2R,4R)-4-amino-2-(4-chlorophenyl)cyclopentyl]-thiomorpholino-methanone:

To a solution of tert-butyl rel-N-[(1R,3R,4R)-3-(4-chlorophenyl)-4-(thiomorpholine-4-carbonyl)cyclopentyl]carbamate (7.0 g, 16.5 mmol, 1 equiv.) in 1,4-dioxane (50 mL) was added HCl (4N in dioxane, 40 mL, 160.0 mmol, 10 equiv.) and the reaction mixture was allowed to stir at room temperature.

The reaction progress was monitored by UHPLC (acidic buffer, gradient conditions A) and full conversion was observed after 20 hours.

The reaction mixture was concentrated in vacuo. The crude was suspended in diethyl ether (100 mL) and stirred for 30 min. The solid was filtered and dried in vacuo, providing the target compound as HCl salt (6.3 g, quant.).

MS (ES-APCI) m/z: [M+H]⁺ calc. for C₁₆H₂₂ClN₂OS⁺ 325.1, found 325.0.

1H NMR (300 MHz, MeOD-d₄, δ in ppm): 7.36-7.32 (overlapping peaks, 4H), 4.03-3.97 (m, 2H), 3.61-3.49 (m, 4H), 3.33-3.32 (m, 1H), 2.62-2.48 (m, 3H), 2.43-2.24 (m, 3H), 2.05-1.96 (m, 2H).

### Example 3: Synthesis of compound ZXR-810, rel(1R,2R,4R)-4-amino-2-(4-chlorophenyl)-N-isopropyl-N-methyl-cyclopentanecarboxamide

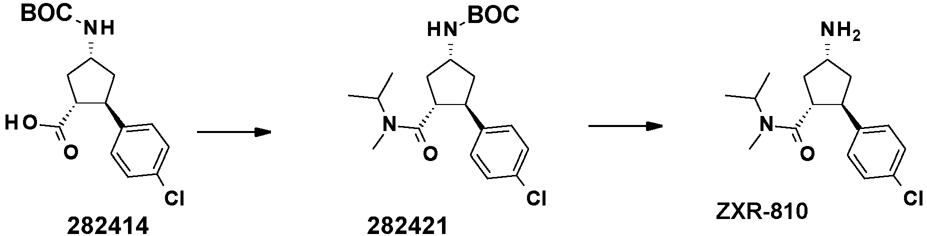

### a) Preparation of compound 282421, tert-butyl rel-N-[(1R,3R,4R)-3-(4-chlorophenyl)-4-[isopropyl (methyl)carbamoyl]cyclopentyl]carbamate:

To a solution of rel-(1R,2R,4R)-4-(tert-butoxycarbonylamino)-2-(4-chlorophenyl)cyclopentanecarboxylic acid (7.0 g, 20.6 mmol, 1.0 equiv.) in dichloromethane (100 mL) was added 1-hydroxybenzotriazole (4.4 g, 30.9 mmol, 1.5 equiv.), 1-Ethyl-3-(3-dimethylaminopropyl)carbodiimide hydrochloride (8.0 g, 41.2 mmol, 2.0 equiv.), diisopropylethylamine (14.4 mL, 82.4 mmol, 4.0 equiv.) and isopropylmethylamine (4.3 mL, 41.2 mmol, 2.0 equiv.) was added and the reaction mixture was allowed to stir at room temperature.

The reaction progress was monitored by UHPLC and full conversion was observed after 20 h. The reaction mixture was washed with ammonium chloride (5x75 mL) and sodium hydrogencarbonate (2x75 mL). The organic phase was dried over MgSO₄ and then concentrated in vacuo. The crude was purified by flash column chromatography (solvent system cyclohexane/methyl tertbutyl ether) to give the title compound (4.9 g, 60%).

MS (ES-APCI) m/z: [M-Boc+H]⁺ calc. for C₁₆H₂₄ClN₂O⁺ 295.1, found 295.3.

1H NMR (300 MHz, CDCl₃, d in ppm; rotamers observed): 7.19 (d, 4H), J=8.4 Hz), 7.08 (d, 4H, J=8.4 Hz), 5.90 (bs, 2H), 4.8 (septet, 1H, J=6.8 Hz, major rotamer), 4.30-4.15 (m, 2H), 3.64 (septet, 1H, minor rotamer, J=6.6 Hz), 3.48-3.37 (m, 2H), 3.12-2.96 (m, 2H), 2.67 (s, 3H, minor rotamer), 2.36 (s, 3H, major rotamer), 2.30-2.22 (m, 2H), 2.12-2.06 (m, 2H), 1.97-1.75 (m, 4H), 1.38 (s, 18H), 0.98 (d, 3H, J=6.6 Hz, minor rotamer), 0.96 (d, 3H, J=6.8 Hz, major rotamer), 0.94 (d, 3H, J=6.8 Hz, major rotamer), 0.98 (d, 3H, J=6.6 Hz, minor rotamer).

### b) Preparation of compound ZXR-810, rel(1R,2R,4R)-4-amino-2-(4-chlorophenyl)-N-isopropyl-N-methyl-cyclopentanecarboxamide:

To a solution of tert-butyl rel-N-[(1R,3R,4R)-3-(4-chlorophenyl)-4-[isopropyl(methyl) carbamoyl]cyclopentyl]carbamate (4.9 g, 12.5 mmol, 1.0 equiv.) in 1,4-dioxane (45 mL) was added HCl (4N in 1,4-dioxane, 31 mL, 124.0 mmol, 10 equiv.) and the reaction mixture was allowed to stir at room temperature.

The reaction progress was monitored by UHPLC (acidic buffer, gradient conditions A) and full conversion was observed after 4 hours.

The precipitated solid was filtered, resuspended in diethyl ether (100 mL) and stirred for 30 min. The solid was filtered and dried in vacuo, providing the target compound as HCl salt (3.6 g, quant.).

MS (ES-APCI) m/z: [M+H]⁺ calc. for C₁₆H₂₄ClN₂O⁺ 295.1, found 295.7.

1H NMR (300 MHz, DMSO-d₆, δ in ppm): 8.4 (bs, 6H - protonated amino group), 7.32 (d, 4H, J=8.4 Hz), 7.25 (d, 4H, J=8.4 Hz), 4.57 (septet, 1H, J=6.7 Hz, major rotamer), 3.96 (septet, 1H, J=6.5 Hz, minor rotamer), 3.86-3.70 (m, 2H), 3.56-3.47 (m, 2H), 3.29 (m, 2H), 2.56 (s, 3H, minor rotamer), 2.49 (s, 3H, major rotamer), 2.43-2.32 (m, 2H), 2.15-2.01 (m, 4H), 1.84-1.75 (m, 2H), 1.02 (d, 3H, J=6.5 Hz, minor rotamer), 0.93 (d, 3H, J=6.7 Hz, major rotamer), 0.83 (d, 3H, J=8.7 Hz, major rotamer), 0.69 (d, 3H, J=6.5 Hz, minor rotamer).

### Construction of Tob1a overexpression zebrafish line

To generate the Tob1a overexpression zebrafish line (myl7:loxP-myc-BFP-loxP-Kusabira Orange-p2A-Tob1a), fertilised eggs were obtained from mating of AB wild type adult zebrafish. A pool of 50 embryos was randomly selected and RNA extraction and cDNA synthesis was performed. The Tob1a coding sequence (CDS) was amplified from cDNA of 48 hpf embryos using the following primers: Forward (Fw): ATGCAGCTTGAAATCCAAGTAG (SEQ ID NO: 1), Reverse (Rv): GTTGGCCATCACTGGCTG (SEQ ID NO: 2). The amplified fragment was then cloned downstream of Kusabira Orange and in frame with the open reading frame (ORF) using Gibson Assembly. The cloning was performed in a pDestTol2pA2AC vector (Kwan et al., 2007) that contained the following elements: (myl7-LoxP-myc-BFP-rabbit β-globin polyA signal-LoxP-p2A-Kusabira Orange-SV40 polyA; cryaa:cerulean), after digestion with Clal and SnaBI (Mercader lab, unpublished). The entire construct was flanked by Tol2 sites to facilitate transgenesis. In this transgenic line, cardiomyocytes express myctag-BFP. Upon Cre-recombination, Kusabira Orange-p2A-Tob1a is expressed specifically in cardiomyocytes. The full name of this transgenic line is Tg(myl7:loxP-myc-BFP-loxP-Kusabira Orange-p2A-Tob1a; cryaa:cerulean). The transgenic line was generated by co-injecting Tol2 mRNA and the plasmid in one-cell stage AB zebrafish embryos. F0 founders were screened and selected based on cerulean expression in the crystallin and BFP expression in cardiomyocytes. Selected F0 founders were crossed with Tg(cmlc2:CreER)^{pd10} and raised to adulthood.

TheTg(cmlc2:CreER)^{pd10} was generated in the following manner: cmlc2:CreER Cre-ERT2 cDNA from pCre-ERT230 was cloned downstream of the 5.1 kb cmlc2 promoter31. A DsRed-Ex cassette controlled by the lens specific α-crystalline promoter was also included (α-cry:DsRed), which enables visual identification of transgenic animals by lens fluorescence32, and was subcloned upstream of the cmlc2:CreER sequences in the opposite orientation. The entire construct was flanked with two copies of the core element of the chicken β-globin insulator (2× core insulator elements) as described in Kikuchi et al., 2010.

### Adult Zebrafish preparation for cardiomyocyte proliferation analysis

Briefly, 9 months old adult animals *Tg(myl7:loxP-myc-BFP-loxP-Kusabira Orange-p2A-Tob1a; cryaa: cerulean); Tg(cmlc2:CreER)^{pd10}* were used.

The CreErT2 is fused to a triple mutant form of the human estrogen receptor, which does not bind to its natural ligand (17 beta-estradiol) at physiological concentrations but to the synthetic estrogen receptor ligand 4-hydroxytamoxifen (OHT). The OHT is used to specifically excise the *IoxP-myc-BFP-IoxP* sequence and allow the expression of *Kusabira Orange-p2A-Tob1a* transgene under the cardiomyocyte promoter *cmlc2.* 36h before cardiac cryoinjury, the synthetic oestrogen receptor 4-hydroxytamoxifen (4OHT) was administered at a concentration of 10 µM in E3 medium overnight (12 hours pulse). 24 hours prior to cardiac injury, 4-OHT medium was washed out and replaced by fresh E3 medium. On day 0, cardiac cryo-injury was performed. At 6 dpi, 20 µl BrdU (2.5 mg/ml) in phosphate buffered saline (PBS) was injected intraperitoneally. At 7 dpi (24 hours BrdU pulse), hearts were collected and analyzed.

### 4-OHT treatment

Before administration, 4-OHT 10 mM stock (dissolved in ethanol) was heated 10 min at 65 °C. 10 µM 4-OHT (Sigma, H7904) was administered to adult zebrafish 36 hours before cardiac cryoinjuries. Treatments were performed overnight (12 hours pulse).

### Zebrafish Cardiac Cryoinjuries

The cardiac ventricular cryoinjury experiments were performed on adult zebrafish following the protocol described (González-Rosa and Mercader, 2012). In summary, the adult fish were anesthetized, and their pericardial cavity was opened to expose the heart. A copper filament, previously cooled in liquid nitrogen, was carefully applied to the ventricular surface of the heart until it thawed. Following the surgical procedure, the animals were revived by gently directing water to their gills using a plastic Pasteur pipette.

### Zebrafish Histological Analysis and imaging

Adult zebrafish were euthanized by immersion in 0.16% tricaine and hearts dissected and processed as described in (González-Rosa and Mercader, 2012). Samples were fixed overnight at 4°C in 2% PFA and included in gelatin following conventional histological procedures. Immunofluorescence of gelatin-embedded 7 µm sections was performed as described in González-Rosa et al., 2011. Briefly, samples were permeabilized with 0.5% Triton X-100 in PBS, blocked in histoblock (5% BSA, 5% goat serum, 20mM MgCl₂) for 2 hours at room temperature, and incubated with primary antibodies in PBS containing 5% BSA overnight at 4°C. Samples were incubated with secondary antibodies at room temperature for 2 hours and incubated with DAPI for 10 minutes. Primary antibodies used were: mouse IgG1 anti-MHC (DSHB F59, 1:20), mouse anti-BrdU (BD PharMingen; 1:250) and rabbit anti-Mef2c (Santa Cruz Biotechnology, C21, sc-313, 1:200) Secondary antibodies were Alexa Fluor 488, 568, 647 (Life Technologies, 1:250). Nuclei were counterstained with DAPI and slides were mounted in Dako Fluorescence Mounting Medium.

### Histological sections imaging

A Zeiss Leica SP8 confocal microscopes were used to image immunostained sections.

### Cardiomyocyte proliferation: immunostaining analysis

3 ventricular sections containing the largest injury areas were imaged per heart. BrdU⁺; MHC⁺, Mef2C⁺ (proliferating cardiomyocytes) and BrdU⁻,MHC⁺,Mef2C⁺ (non proliferating cardiomyocytes) were counted using imaged software from the whole ventricle. The cardiomyocyte proliferation index represents the number of proliferating cardiomyocytes in relation to the total number of cardiomyocytes and is assessed in the border zone, close to the site of injury. The final cardiomyocyte proliferation index per animal was calculated as the average of the three sections.

### Cell culture of human induced pluripotent stem-cell derived cardiomyocytes (hiPSC-CMs)

Human Induced pluripotent stem-cell derived (iPS-CMs) cardiomyocytes (iCell^{®} Cardiomyocytes, Cat. No. 11713) were purchased from FUJIFILM Cellular Dynamics. hiPSC Cardiomyocytes were seeded into 96 well plates coated with 0,1% gelatin (STEMCELL tech, 07903) with iCell Cardiomyocytes plating medium (Fujifilm, M1001). After 48 hours, the medium was changed to iCell Cardiomyocytes maintenance medium (Fujifilm, M1003) and cells were cultured for 2 weeks with media replacement every 48-72 hours. Cells were maintained in a humidified incubator (Thermo Scientific,HERACELL 150i) in a 95% air 5% CO₂ environment at 37 °C

After 8 days, hiPSC-CMs started beating spontaneously and these cells are known to show molecular, electrophysiological, metabolic, mechanical, and ultrastructural properties similar to primary human cardiomyocytes (Karakikes et al., 2015). We used this cellular model to evaluate proliferation (CellTiterGLo and Ki67 marker), migration (Wound healing assay), and differentiation markers (cardiac troponin T: cTnT) in response to the compounds ZXR-807, ZXR-810, ZXR-081, ZXR-090.

### Tob1 knockdown using small interferent silencing RNAs

H9C2 and MCF-7 cells were plated at 100,000 cell/ml in DMEM + 10% FBS in p24 wells plate and incubated at 37°C for 24 hours. We used Silencer^{®} Select siRNAs to knock down Tob1 (s139396 and s139397 from ThermoFisher Scientific).

The siRNAs (final concentration 90 nmol/l) were transfected using Lipofectamine 3000 (ThermoFisher, L3000001) according to the manufacturer's instructions. Briefly, siRNA (final concentration 90 nmol/l) and Lipofectamine 3000 were firstly diluted separately in Opti-DMEM medium (Gibco^{™}; Life Technologies, Grand Island, NY, USA) without antibiotics or serum, and incubated together for 15 min. The lipofectamine-siRNA complex was added to the cells and incubated for 4 hours then the transfection medium was changed to DMEM+10% FBS and gene expression was analyzed at 48 hours.

### Tob1 knockout in H9C2 cells using CRISPR/Cas9 system

100.000 H9C2 cells were plated in a 24 well plate day before transfection with lipofectamine CRISPRMAX^{™} (Thermofisher, CMAX00001), to obtain a 70% of confluence. The CRISPRMAX^{™} assay was performed following the manufacturer's instructions. Briefly Cas9 nuclease/gRNA RNP complexes were prepared by combining the following components , 3,85 µ! of 2 µM Alt-R S.p. Cas9 Nuclease v3 (1081059, IDT)1; 2,25 µ! of 2 µM sgRNAs (Tob1 sgRNA1: AAAAATGTTGACACGTCTCC (SEQ ID NO: 3) and Tob1 sgRNA2: GTTAAAGGCTGAGTGGACCG (SEQ ID NO: 4), negative control: GCACUACCAGAGCUAA (SEQ ID NO: 5), Synthego), 2,5 µ! Cas9 plus reagent in 16,65 µl Opti-MEM^{™}(Gibco^{™}; Life Technologies, Grand Island, NY, USA) in tube 1 and 1,5 µl lipofectamine CRISPRMAX^{™} in 25 µ! in Opti-MEM^{™} in tube 2. The Cas9/sgRNA ribonucleoprotein (RNP) complex was obtained by incubating the mix of tub1 and 2 for 10 minutes at room temperature. 50 µL of the RNP complex was added to cells in 450 µL of fresh high glucose DMEM (ATCC, 30-2002), supplemented with 10 % FBS and without antibiotics and cells were incubated with the RNP complex for 48 h at 37 °C.

48h post transfection, cells were washed with Phosphate Buffered Salt solution and detached with trypsin-EDTA. To confirm gene editing success, DNAs of H9C2 cells of Tob1 and negative control transfected cells were extracted with COBO SCIENTIFICXtract reagent (Cobo Scientific, CX0150) and amplified by PCR (oneTaqQuick, NEB,174M0486L) with the Tob1 primers (Fw: TTTGTTTCTCTGCCACAAACTGA (SEQ ID NO: 6) and Rv: CTGAGGTTAAGGGGGCTGTC (SEQ ID NO: 7), IDT)

DNA was purified directly from PCR reactions with the QIAquick PCR Purification Kit (Qiagen, Cat. No. / ID: 28104) or from agarose bands with the QIAquick Gel Extraction Kit (Qiagen, Cat. No. / ID: 28704) following the manufacturer's instructions. In both cases, DNA was finally eluted with 11 µl of nuclease-free water and 0.4 µl of the 100 µM of the Tob1 forward primer was added to the mix. Samples were sent for Sanger sequencing to StabVida, LDA (Caparica, Portugal).

### Cell proliferation assays

### 1) MTT reduction assay and Tob1 Knockdown

3-(4,5-dimethylthiazol-2-yl)-2,5-diphenyltetrazolium bromide, MTT (Sigma, M5655-500MG) assay was used as a surrogate to evaluate cell number and cell proliferation in H9C2 and MCF-7 cells. MTT is a colorimetric assay that measures cell metabolic activity in viable cells, resulting from the activity of mitochondrial dehydrogenases which cleave the tetrazolium ring of the yellow tetrazolium salt MTT, yielding formazan crystals. The protocol was designed to align Tob1 peak decrease in mRNA expression levels (48-96 hours post-transfection) with the period of exponential growth of the cells (24-72 hours post-replating).

MCF-7 cells and H9C2 cells were seeded at 100,000 cells/well in 24-well in DMEM + 10% FBS plates to reach 80-90% at time of transfection. MCF-7 and H9C2 cells were transfected 24 hours post-plating with Silencer^{®} Select siRNAs to knock down Tob1. 48 hours post-transfection, cells were lifted and plated at 10000 (MCF-7) and 8000 cells/well (H9C2) in 96-well plates and grown for 144 hours. At day 6 (144 hours), 0,5 mg/ml MTT was added and incubated for 3 hours, producing formazan crystals that were solubilized with SDS 20%+ HCl 0.02N. 24 hours later, absorbance was measured at 590 nm with the spectrophotometer FLUOstar Omega plate reader (BMG LabTech).

### 2) MTT assay in cells treated with the compounds of the invention

Both Tob1 expressing and Tob1 knockout H9C2 cells were seeded at 8000 cells/well in 96-well plates. 48 hours later, cells were treated with 1.28 nM; 6.4 nM; 32 nM, 160nM, 800 nM, 4 µM and 20 µM ZXR-807, ZXR-810; ZXR-081 and ZXR-090 and cell proliferation was measured 144 hours post-treatment using the MTT assay as described above.

### 3) CellTiter-Glo^{®} assay in hiPSC-CMs

CellTitler-Glo^{®} (Promega, G7570) was used to measure the activity of the compounds of the invention in triggering proliferation in hiPSC-CMs. The CellTiter-Glo^{®} Luminescent Cell Viability Assay, similar to the MTT assay but with higher sensitivity, determines the number of metabolically active and viable cells, and is based on the quantification of ATP levels in the cells.

hiPSC-CMs were seeded at 7000 cells/well in 96-well plates and treated with the compounds of the invention at 8; 40 and 200nM in a 0.3% DMSO containing iCell Cardiomyocyte medium and a control treatment or vehicle with 0.3% DMSO in iCell cardiomyocyte medium. hiPSC-CMs proliferation was measured after 144h (6 days) , and 336 (14 days) of treatment. At each time point, 100 µL of CellTiter Glo (ratio 1:1 CellTiter Glo: cell culture medium ) was added to each well containing 100 µL iCell Cardiomyocyte medium, following the manufacturer's instructions. Luminescence was read in Varioskan (Thermo Fisher Scientific), 30 minutes after adding the reagent.

### Cell migration (wound healing) assay in H9C2 cells and hiPSC-CMs

H9C2 cells were seeded at 100,000 cells per well in 24-well plates in a low containing serum medium (DMEM high glucose+ 1% FBS) to favor migration over proliferation. 2 hours after seeding, cells were scraped with a 1000 µL tip in the center of the well and treated with 100nM ZXR-807, ZXR-810, ZXR-081 and ZXR-090 and 0,3% DMSO (vehicle) in control conditions. Plates were imaged with an inverted microscope (Leica, DMI6000B) right after generating the wound (0 h) and 28 hours post-scratch.

hiPSC-CMs cells were seeded at 25,000 cells per well in 96 wells plates. 168 hours after seeding, cells were scraped with a 200 µL tip and treated with 8, 40 and 200nM ZXR-807, ZXR-810, ZXR-081 and ZXR-090. Plates were imaged with an inverted microscope (Leica, DMI6000B) right after generating the wound (0 h) and 144 hours post-scraping.

All images were analyzed using the Fiji software (Schindelin et al., 2012) and the amount of cellular migration was determined by calculating the wound area at 0h and 28 hours or 144 hours and normalized to control vehicle-treated cells (0,3% DMSO).

### Immunocytochemistry and fluorescent microscopy: proliferation and differentiation markers in hiPSC-CMs

Following the migration assay, the medium was aspirated from the 96-well plates, and cells were washed twice with phosphate buffered saline solution (PBS) 1x (Fisher bioreagents, BP399-4). Cells were fixed with 4% paraformaldehyde (Sigma, 158127) for 10 minutes at room temperature and washed 3 times with PBS 1x. Cells were permeabilized with Triton^{™} X-100, 0.03% (Merck, T8787) and and blocked with 0.5% Goat serum (ThermoFisher, 16210064) in PBS 1x, during 1 hour at room temperature. The primary antibodies Ki67 rabbit antibody 1/100 (Santacruz, sc-23900), and cardiac Troponin (cTnT mouse antibody 1/100 (ThermoFisher, MA5-12960) were diluted in the following buffer: Triton^{™} X-100 0.03% and 1% Bovine serum albumin (BSA) 10% (nzytech, MB04602) and PBS 1x. hiPSC-CMs were incubated with the primary antibodies overnight at 4°C, washed three times with PBS 1x and incubated with the secondary antibodies goat anti-mouse or anti -rabbit coupled to Alexa fluorescent dyes (company). DAPI 1/1000 (Merck, D9542-5MG) was incubated for 20 min at room temperature to visualize nuclei. Cells were washed out 3x with PBS and kept in PBS at 4°C.

Preparations were examined on a fluorescent inverted microscope Leica, DMI6000B using 20x dry objectives from Leica. Images were analyzed with the open source image processing package Fiji to quantify Nuclear Ki67 fluorescent intensity and cellular cardiac troponin T (cTnT) normalized to the number of nuclei in the field of view

### Quantitative Real-Time-PCR

In order to evaluate 1) efficiency of Tob1 knockdown in MCF-7 and H9C2 cells and 2) expression levels of proliferation markers (Pcna, Cdk1 and Ccnb1) in H9C2 cells treated with the compounds of the invention, H9C2 cells were harvested by trypsinization and centrifuged at 300 x *g* for 5 min. Total mRNA was isolated using the Maxwell 16 LEV simplyRNA Cells Kit or Tissue Kit (Promega, Madison, WI) following the manufacturer's instructions. Nanodrop ND-1000 Spectrophotometer (Thermo Fisher Scientific, Waltham, MA) was used to quantity the amount of RNA extracted. cDNA synthesis was performed from 300 ng of total RNA with the SuperScript IV First-Strand Synthesis System (Thermo Fisher Scientific, Waltham, MA).

Real-time PCR was conducted using LightCycler 480 SYBR Green I Master on a LightCycler 480 thermal cycler (Roche, Basel, Switzerland). The expression levels of the indicated genes were calculated by the conventional 2^{-DDCt} method, with normalization to housekeeping gene glyceraldehyde-3-phosphate dehydrogenase (GAPDH). Forward and reverse primers sequences are listed in Table 1:

**Table 1**

| Genes | Forward primer (5'- 3') | Reverse primer (5'- 3') |
|---|---|---|
| Cdk1 | GATCTGTAAGGACCAGCTCAC (SEQ ID NO: 8) | CCATAAGTCCCTTCTCCGATTT (SEQ ID NO: 9) |
| Ccnb1 | GCCAGAGGTTAGGGTGTCTT (SEQ ID NO: 10) | AGTTCTCGATCTCAGCAGGG (SEQ ID NO: 11) |
| Gapdh | ACTCCCATTCTTCCACCTTTG (SEQ ID NO: 12) | CCCTGTTGCTGTAGCCATATT (SEQ ID NO: 13) |
| Tob1 | GAACCACAGCAGCCATTTGTG (SEQ ID NO: 14) | AACCACATGGTTCACCCTCG (SEQ ID NO: 15). |

### RESULTS

### 1. Tob1 as a relevant target in cardiac regeneration in vivo.

### Zebrafish Tob1 OE system displays decreased proliferation

To validate the role of *tob1* in cardiac regeneration, *tobla* was overexpressed in zebrafish cryoinjured hearts by using a cardiomyocyte-specific, temporally inducible genetic overexpression system. In adult control hearts, a high rate of proliferation of cardiomyocytes in the myocardial area, reflected by the high density of BrdU-positive cells, was detected. This result was expected in the zebrafish-specific endogenous cardiac regeneration process at 7 days after injury. However, *tobla* overexpression caused a significant reduction in cardiomyocyte proliferation, as shown by a decrease in the proliferation index, falling to half the value observed in the control condition (Fig. 1A). Decrease in proliferative cardiomyocytes in the Tob1 OE zebrafish hearts following injury is also associated with a reduction in the density of proliferating, BrdU positive cardiomyocytes (number of cells per area) (Fig. 1B). These results suggest that Tob1 overexpression inhibits the proliferation of adult zebrafish cardiomyocytes and leads to a reduction in the number of cardiomyocytes following injury.

### 2. Tob1 represses proliferation and migration in relevant cell types.

The inventors validated the effect of Tob1 inhibition in two different cell lines: MCF7, a human breast cancer cell line, which validates that Tob1 is relevant in human tissues; and H9C2, a rat cardiomyoblast cell line, which validates that Tob1 plays a role in a cell line derived from cardiac tissue.

The relevance of Tob1 was first evidenced by its expression in MCF-7 and H9C2 cells at perinuclear and nuclear subcellular locations. This observation derived from fluorescent microscopy images of both cell types, where Tob1 was detected with fluorescent immunohistochemistry by the use of a mouse derived antibody against Tob1 and a secondary green fluorescent antibody against the mouse antibody epitope, nuclei were marked with the DNA specific probe DAPI.

Then, to confirm that Tob1 was involved in the processes of cellular proliferation, Tob1 mRNA expression was depleted by more than 70%, by siRNA knockdown in MCF-7 and H9C2 cells (Fig. 2A and Fig. 2B). In both cases, the Tob1 promoted a significant increase in proliferation of respectively 37% in MCF-7 cells and 10% in H9C2 cells (Fig. 2A and Fig. 2B, left panel). Furthermore, we confirmed that complete depletion of Tob1 in H9C2 cells by CRISPR/Cas9 knockout technology, promoted an increase in proliferation of 15% (Fig. 2C). H9C2 Tob1 knockout cells also displayed a notable increase in cell migration, as shown by the migration-wound healing assay and the higher capacity of these cells to repopulate the injured region (Fig. 2D).

### 3. The compounds of the invention are potent and selective Tob1 inhibitors

Once lead compounds were selected, their efficacy, potency and selectivity at promoting proliferative responses, were evaluated *in vitro* in rat cardiomyoblasts (H9C2 cells) using MTT assay. H9C2 cells were treated with vehicle 0,3% DMSO and increasing concentrations of Tob1 inhibitors: ZXR-807, ZXR-810, ZXR-081 and ZXR-090, (1,28 nM to 20 µM), followed by the assessment of proliferation, using the MTT assay, after cells were left to grow for 144 hours (6 Days).

All four Tob1 inhibitors triggered increases in proliferation between 26 to 36% above control values, with maximal biological responses observed at low micromolar concentrations (4 µM) as shown in Table 1.

**Table 1: Lead efficacy (%) at triggering proliferation in H9C2 cells.**

| compound ID | concentration microMolar | H9C2 % Proliferation (normalized to control) | SEM |
|---|---|---|---|
| ZXR-807 | 4 | 27,4 | 4,7 |
| ZXR-810 | 4 | 26.5 | 2,1 |
| ZXR-081 | 4 | 36,6 | 5,5 |
| ZXR-090 | 4 | 26,6 | **2,4** |

Tob1 inhibitors selectivity was assessed by comparing dose-responses of ZXR-807, ZXR-810, ZXR-081 and ZXR-090 in H9C2 control cells and H9C2 Tob1 KO cells where Tob1 was depleted by CRISPR/Cas9 technology. Compounds have a good selectivity at targeting Tob1, especially ZXR-807 and ZXR-081 which activate proliferation in H9C2 cells with EC50< 60,000 than EC50 in Tob1 KO H9C2 cells, meaning that these two Tob1 inhibitors are highly selective for Tob1 (Table 2 and Fig. 3). ZXR-810 and ZXR-090 have lower selectivity at Tob1 but still activate proliferation in H9C2 cells with EC50 values < 200-300 than in Tob1 KO cells. Table 2: Comparison of EC50 values in response to lead compounds in Tob1 and Tob1 depleted (KO) H9C2 cells.

| compound ID | Tob1 EC50 (nM) | Tob1 KO EC50 (nM) |
|---|---|---|
| ZXR-807 | 1.16E-04 | 8.28E+01 |
| ZXR-810 | 6.20E-01 | 6.28E+01 |
| ZXR-081 | 1.48E-04 | 1.77E+02 |
| ZXR-090 | 1.28E-01 | 6.28E+01 |

The dose-response to Tob1 inhibitors showed that all four compounds ZXR-807, ZXR-810, ZXR-081 and ZXR-090 displayed high potencies (EC50) and produced significant increases in proliferation of H9C2 cells at low nanomolar concentrations (Table 2 and Fig. 3).

### 4. The compounds of the invention activate cell cycling genes expression

The compounds of the invention displayed a strong capacity to promote cellular proliferation of rat cardiomyoblasts. Indeed, increased proliferation is associated with the activation of cell cycle gene transcription, which controls cell cycle progression through G1-S-G2-M phases. In line with that, the Tob1 inhibitors ZXR-807, ZXR-810, ZXR-081 and ZXR-090 promote the transcriptional activation of a key marker of the G2/M transition phase Cdk1, with about 3-fold increase in the case of ZXR-081 and ZXR-090 while Ccnb1 expression, which acts together with Cdk1 to regulate early mitosis events, is more moderately increased with ZXR-807, ZXR-810 and ZXR-090 (Fig. 4).

### 5. The compounds of the invention activate cell migration

In addition, these molecules show a remarkable increase in cell migration (Fig. 5). We scrapped H9C2 cells to generate a wound in the center of the well and measured how cells migrated towards the injured region after 28 hours in control conditions (vehicle) and in the presence of the Tob1 inhibitors. All compounds promoted an increase in migration of more than 50% when compared to vehicle treated cells. This is an important cell process, given that migration has been shown to be coordinated with proliferation to ensure, not only the renewal of lost cardiomyocytes, but also their migration to the site of injury to replace damaged cells and, therefore, sustain cardiac regeneration.

### 6. Tob1 inhibitors promote proliferation and migration of human iPSC-derived cardiomyocytes

### 6.1. The compounds of the invention trigger proliferation and inhibit differentiation of hiPSC-CMs

The inventors tested the compounds of the invention at a concentration of 8nM and found that ZXR-807, ZXR-810 and ZXR-081 increased the proliferation rate of hiPSC cardiomyocytes by 20-28% at 144 hours (6 days) and by 40% for ZXR-807 and ZXR-810 after 336 hours (14 days) of treatment (Fig. 6A). ZXR-081 has a transitory effect on proliferation increase, peaking at 144 hours and disappearing by 336 hours post treatment. The nuclear signal intensity of Ki67, a known marker for proliferative cells used in clinics, was assessed by immunostaining in hiPSC cardiomyocytes in cells treated with Tob1 inhibitors (Fig 6B), both close to the scratch or wound area and further away from the wound. Ki67 is expressed in cycling cells during the G1, S; G2 and M phases but not in G0 (quiescent cells). ZXR-081 significantly increased nuclear Ki67 signal intensity in hiPSC cardiomyocytes, close to the scratch while ZXR-807, ZXR-810 and ZXR-081 inhibitors increased nuclear Ki67 expression away from the scratch, reflecting the concomitant increase in proliferation in these cells.

On the other hand, hiPSCs, when differentiated to cardiomyocytes, express typical mature cardiac markers such as cardiac troponin T (cTnT) In response to Tob1 inhibitors (144 hours post-treatment), differentiated hiPSC cardiomyocytes show reduced expression of the differentiation marker cTnT, especially close to the scratch-wound region (Fig. 7). Decreased cTnT expression, following Tob1 inhibition, supports the idea that the cells dedifferentiate to an intermediate state where sarcomeres are partially disassembled and which is necessary for the cells to reenter the cell cycle and proliferate (Jopling et al., 2011).

### 6.2. Lead compounds promote migration of hiPSC-CMs

As shown before, cell migration is a crucial component of cardiac regeneration. Interestingly, all lead compounds ZXR-807, ZXR-810, ZXR-081 and ZXR-090 activate cell migration in the low nanomolar concentration range, (8 nM) as shown in Fig. 8.

In short, our results demonstrate that Tob1, transducer of ERBB2 1, is involved in the control of cardiomyocyte proliferation and migration and represents a new and promising target for heart regeneration *in vitro* and *in vivo.*

The inventors successfully identified new bioactive small molecules, inhibitors of Tob1-Cnot7 interaction, that can promote proliferation, migration and regenerative processes in mammal cardiomyocytes. Indeed, small molecules offer a valuable and necessary alternative to stem cell therapy and palliative treatments to improve cardiac function following myocardial infarction.

### Bibliographic references

Bai Y, Tashiro S, Nagatoishi S, Suzuki T, Yan D, Liu R, Tsumoto K, Bartlam M, Yamamoto T. Structural basis for inhibition of the Tob-CNOT7 interaction by a fragment screening approach. Protein Cell. 2015 Dec;6(12):924-8. doi: 10.1007/s13238-015-0225-6. PMID: 26518565; PMCID: PMC4656213.
Carbone RG, Monselise A, Bottino G, Negrini S, Puppo F. Stem cells therapy in acute myocardial infarction: a new era? Clin Exp Med. 2021 May;21(2):231-237. doi: 10.1007/s10238-021-00682-3. Epub 2021 Jan 23. PMID: 33484381; PMCID: PMC8053645.
Doidge R, Mittal S, Aslam A, Winkler GS. The anti-proliferative activity of BTG/TOB proteins is mediated via the Caf1a (CNOT7) and Caf1b (CNOT8) deadenylase subunits of the Ccr4-not complex. PLoS One. 2012;7(12):e51331. doi: 10.1371/journal.pone.0051331. Epub 2012 Dec 7. PMID: 23236473; PMCID: PMC3517456.
González-Rosa JM, Martin V, Peralta M, Torres M, Mercader N. Extensive scar formation and regression during heart regeneration after cryoinjury in zebrafish. Development. 2011 May;138(9):1663-74. doi: 10.1242/dev.060897. Epub 2011 Mar 23. PMID: 21429987.
González-Rosa JM, Mercader N. Cryoinjury as a myocardial infarction model for the study of cardiac regeneration in the zebrafish. Nat Protoc. 2012 Mar 29;7(4):782-8. doi: 10.1038/nprot.2012.025. PMID: 22461067.
Heallen TR, Kadow ZA, Kim JH, Wang J, Martin JF. Stimulating Cardiogenesis as a Treatment for Heart Failure. Circ Res. 2019 May 24;124(11):1647-1657. doi: 10.1161/CIRCRESAHA.118.313573. PMID: 31120819; PMCID: PMC6534162.
Hong CC. The grand challenge of discovering new cardiovascular drugs. Front Drug Discov (Lausanne). 2022;2:1027401. doi: 10.3389/fddsv.2022.1027401. Epub 2022 Sep 23. PMID: 37123434; PMCID: PMC10134778.
Jopling, C., Boue, S. & Belmonte, J. Dedifferentiation, transdifferentiation and reprogramming: three routes to regeneration. Nat Rev Mol Cell Biol 12, 79-89 (2011). https://doi.org/10.1038/nrm3043.
Karakikes I, Ameen M, Termglinchan V, Wu JC. Human induced pluripotent stem cell-derived cardiomyocytes: insights into molecular, cellular, and functional phenotypes. Circ Res. 2015 Jun 19;117(1):80-8. doi: 10.1161/CIRCRESAHA.117.305365. PMID: 26089365; PMCID: PMC4546707.
Kikuchi K, Holdway JE, Werdich AA, Anderson RM, Fang Y, Egnaczyk GF, Evans T, Macrae CA, Stainier DY, Poss KD. Primary contribution to zebrafish heart regeneration by gata4(+) cardiomyocytes. Nature. 2010 Mar 25;464(7288):601-5. doi: 10.1038/nature08804. PMID: 20336144; PMCID: PMC3040215.
Kwan KM, Fujimoto E, Grabber C, Mangum BD, Hardy ME, Campbell DS, Parant JM, Yost HJ, Kanki JP, Chien CB. The Tol2kit: a multisite gateway-based construction kit for Tol2 transposon transgenesis constructs. Dev Dyn. 2007 Nov;236(11):3088-99. doi: 10.1002/dvdy.21343. PMID: 17937395.
Salari N, Morddarvanjoghi F, Abdolmaleki A, Rasoulpoor S, Khaleghi AA, Hezarkhani LA, Shohaimi S, Mohammadi M. The global prevalence of myocardial infarction: a systematic review and meta-analysis. BMC Cardiovasc Disord. 2023 Apr 22;23(1):206. doi: 10.1186/s12872-023-03231-w. PMID: 37087452; PMCID: PMC10122825.
Schindelin J, Arganda-Carreras I, Frise E, Kaynig V, Longair M, Pietzsch T, Preibisch S, Rueden C, Saalfeld S, Schmid B, Tinevez JY, White DJ, Hartenstein V, Eliceiri K, Tomancak P, Cardona A. Fiji: an open-source platform for biological-image analysis. Nat Methods. 2012 Jun 28;9(7):676-82. doi: 10.1038/nmeth.2019. PMID: 22743772; PMCID: PMC3855844.
Winkler GS. The mammalian anti-proliferative BTG/Tob protein family. J Cell Physiol. 2010 Jan;222(1):66-72. doi: 10.1002/jcp.21919. PMID: 19746446.

## Claims

1. A compound of formula (I), solvate, stereoisomer or salt thereof: 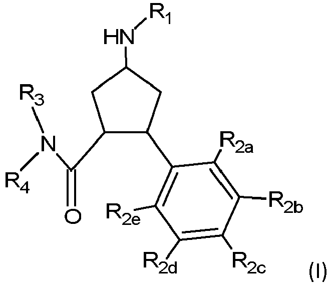 wherein:
R₁ represents H; (C₁-C₁₀)alkyl; (C₁-C₁₀)alkyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; (C₂-C₁₀)alkenyl; (C₂-C₁₀)alkenyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; (C₂-C₁₀)alkynyl; (C₂-C₁₀)alkynyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; -C(O)Rs, and -C(O)OR₆;
R₂ₐ to R₂ₑ are the same or different and represent hydrogen, hydroxyl, or halogen;
R₃ and R₄ are the same or different and represent (C₁-C₁₀)alkyl; (C₁-C₁₀)alkyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; (C₂-C₁₀)alkenyl; (C₂-C₁₀)alkenyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; (C₂-C₁₀)alkynyl; (C₂-C₁₀)alkynyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; or, alternatively,
R₃ and R₄ form, together with the N heteroatom to which they are attached, a 5-6 membered heterocycle including a second heteroatom selected from N, O or S;
R₅ and R₆ represent H; (C₁-C₁₀)alkyl; (C₁-C₁₀)alkyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; (C₂-C₁₀)alkenyl; (C₂-C₁₀)alkenyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; (C₂-C₁₀)alkynyl; (C₂-C₁₀)alkynyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; and
"heterocycle" means a ring system having one or two rings, each one of the rings:
- being saturated, partially saturated or aromatic, and
- consisting of 5 or 6 members, wherein one of the members corresponds to the N atom to which R₃ and R₄ are attached, and the remaining members are selected from CRₓ, C(Rₓ)₂, NR_{y}, N, O, or S; Rₓ is selected from hydrogen, (C₁-C₁₀)alkyl optionally substituted with one or more Z substituents; (C₂-C₁₀)alkenyl optionally substituted with one or more Z substituents; or (C₂-C₁₀)alkynyl optionally substituted with one or more Z substituents; and R_{y} is selected from hydrogen; (C₁-C₁₀)alkyl optionally substituted with one or more Z substituents, (C₂-C₁₀)alkenyl optionally substituted with one or more Z substituents, or (C₂-C₁₀)alkynyl optionally substituted with one or more Z substituents; and Z is selected from the group consisting of: (C₁-C₁₀)alkyl; -O-(C₁-C₁₀)alkyl; (C₁-C₁₀)haloalkyl; -O-(Cₐ-C₁₀)haloalkyl; (C₂-C₁₀)alkenyl; (C₂-C₁₀)alkynyl; and halogen.

2. The compound of formula (I) of claim 1, wherein R₂ₐ to R₂ₑ are the same or different and represent hydrogen or halogen.

3. The compound of formula (I) of any one of the preceding claims, wherein one of R₂ₐ to R₂ₑ is halogen and the others are hydrogen.

4. The compound of formula (I) of any one of the preceding claims, wherein R₁ represents -H or C(O)R₆.

5. The compound of formula (I) of any one of the preceding claims, wherein R₆ represents H; (C₁-C₁₀)alkyl; or (C₁-C₁₀)alkyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; particularly, R₆ represents H or (C₁-C₁₀)alkyl.

6. The compound of formula (I) of any one of the preceding claims, wherein R₃ and R₄ are the same or different and represent (C₁-C₁₀)alkyl; or (C₁-C₁₀)alkyl substituted with one or more groups selected from: -OH, -CN, halogen, -O-(C₁-C₁₀)alkyl, -NH₂, (C₁-C₁₀)alkyl, (C₂-C₁₀)alkenyl, or (C₂-C₁₀)alkynyl; particularly, R₆ represents H or (C₁-C₁₀)alkyl.

7. The compound of formula (I) of any one of the claims 1-5, wherein R₃ and R₄ form, together with the N heteroatom to which they are attached, a 6-membered heterocycle consisting of one ring having 6 members, wherein one of the members is the N atom to which R₃ and R₄ are attached, another of the members is a second heteroatom selected from O or S, and the remaining members are C(Rₓ)₂.

8. The compound of formula (I) which is selected from the group consisting of:
rel-[(1R,2R,4R)-4-amino-2-(4-chlorophenyl)cyclopentyl]-thiomorpholino- methanone;
rel(1R,2R,4R)-4-amino-2-(4-chlorophenyl)-N-isopropyl-N-methyl- cyclopentanecarboxamide;
rel-[(1R,2R,4R)-4-amino-2-(4-chlorophenyl)cyclopentyl]-morpholino-methanone hydrochloride;
rel-N-[(1R,3R,4R)-3-(4-chlorophenyl)-4-(morpholine-4-carbonyl)cyclopentyl] acetamide;
and any salt thereof.

9. A pharmaceutical composition comprising the compound of formula (I), as defined in any one of the preceding claims 1-8, together with one or more pharmaceutically acceptable excipients and/or carriers.

10. A topical cosmetic composition comprising the compound of formula (I), as defined in any one of the preceding claims 1-8, together with one or more cosmetically acceptable excipients and/or carriers.

11. A compound of formula (I), solvate, stereoisomer or salt thereof, according to any one of the preceding claims 1-8, for use in therapy.

12. A compound of formula (I), solvate, stereoisomer or salt thereof, according to any one of the preceding claims 1-8, or a pharmaceutical composition according to claim 9, for use in repairing or regenerating cell tissue, particularly cardiac cell tissue.

13. A compound of formula (I), solvate, stereoisomer or salt thereof, according to any one of the preceding claims 1-9, or a pharmaceutical composition according to claim 9, for use according to claim 12, wherein the compound or composition inhibits TOB1 activity and promotes cellular proliferation.

14. A TOB1 inhibitor for use in in the prevention, repairment or regeneration of a cardiac tissue injury in a mammal by inducing the proliferation and/or migration of cardiomyocytes.

15. Cosmetic use of a compound of formula (I), stereoisomer or salt thereof, according to any one of the preceding claims 1-8, or cosmetic composition according to claim 9, for the skin regeneration or repairment.
